# EUROPEAN PATENT APPLICATION

(11) **EP 4 556 471 A1**
(43) Date of publication of application: **21.05.2025**
(21) Application number: 23839957.0
(22) Date of filing: 12.07.2023
(51) Int. Cl.: C07D 405/04, C07D 409/04, C07D 403/04, C07D 409/14, C07D 405/14, H10K 85/60, H10K 50/11

(54) **ORGANIC COMPOUND AND ORGANIC ELECTROLUMINESCENT DEVICE USING SAME**

(30) Priority: 13.07.2022 KR 20220086403
(71) Applicant: Solus Advanced Materials Co., Ltd., Iksan-si, Jeollabuk-do 54584 (KR)
(72) Inventor: BAE, Hyungchan, Yongin-si, Gyeonggi-do 16858 (KR); SIM, Jaeyi, Yongin-si, Gyeonggi-do 16858 (KR); CHO, Areum, Yongin-si, Gyeonggi-do 16858 (KR)
(74) Representative: Office Freylinger
(86) International application number: PCT/KR2023/009937
(87) International publication number: WO 2024/014866

(57) **Abstract**

The present invention relates to a novel organic compound with excellent carrier transport ability, light emitting ability, and thermal stability, and an organic electroluminescent device that comprises the compound in at least one organic material layer and thus has improved characteristics such as improved luminous efficiency, driving voltage, and lifespan.

## Description

### Technical Field

The present invention relates to a novel organic compound and an organic electroluminescent device using the same and, more specifically, to a novel compound with excellent electron transporting ability, and an organic electroluminescent device exhibiting improved characteristics, such as luminous efficiency, driving voltages, and lifespan, by containing the novel compound in one or more organic layers.

### Background Art

In studies on organic electroluminescent devices, which have continued from Bernanose's observation on organic thin film light emission in the 1950s to blue electroluminescence using an anthracene single crystal in 1965, Tang proposed an organic electroluminescent device with a lamination structure including functional layers of a hole layer and an emissive layer. Thereafter, in order to manufacture organic electroluminescent devices having high efficiency and long lifespan, the introduction of each specific organic layer into an element has been performed, and specialized materials used therein have been developed.

In an organic electroluminescent device, upon the application of voltage between two electrodes, holes from an anode and electrons from a cathode are injected into organic layers. The injected holes and electrons combine with each other to form excitons, and the excitons fall down to the ground state to emit light. Particularly, materials used for the organic layers may be classified into light emission materials, hole injection materials, hole transport materials, electron transport materials, electron injection materials, and the like according to the function thereof.

Materials for forming an emissive layer of the organic electroluminescent device may be classified into blue, green and red light emission materials according to the color of light emission. Additionally, yellow and orange light emission materials may be used as light emission materials for displaying better natural colors. Additionally, host/dopant-based light emission materials may be used as light emission materials to increase color purity and improve luminous efficiency through energy transfer. Dopant materials may be classified into fluorescent dopants using organic materials and phosphorescent dopants using metal complex compounds containing heavy atoms, such as Ir and Pt. These phosphorescent materials can theoretically improve the luminous efficiency up to four times compared to fluorescent materials, so attention is focused on phosphorescent host materials as well as phosphorescent dopants. Until today, NPB, BCP, Alq₃, and the like have been widely known as materials for use in a hole injection layer, a hole transport layer, an electron transport layer, and an electron injection layer, and as for light emission materials, anthracene derivatives have been reported as fluorescent dopant/host materials. Particularly, as for phosphorescent materials having a large advantage in terms of efficiency improvement among light emission materials, metal complex compounds containing Ir, such as Firpic, Ir(ppy)₃, and (acac)Ir(btp)₂, are used as blue, green, and red dopant materials. Until today, CBP shows excellent properties as a phosphorescent host material.

However, conventional light emission materials have advantages in terms of light emission characteristics, but are not satisfactory in terms of lifespan of organic electroluminescent devices due to low glass transition temperatures and very poor thermal stability. Accordingly, there is a need to develop light emission materials with excellent performance.

### Disclosure of Invention

### Technical Problem

The present invention has been made to solve the above-mentioned problems, and an aspect of the present invention is to provide a novel compound, which can be used as a material for an organic layer of an organic electroluminescent device, due to excellent hole, electron injection and transporting ability, light emitting ability.

Another aspect of the present invention is to provide an organic electroluminescent device exhibiting a low driving voltage, high luminous efficiency, and improved lifespan by containing the above-described novel compound.

Other purposes and advantages of the present invention will be clarified by following detailed description and claims.

### Solution to Problem

To achieve the above objectives, the present invention provides a compound represented by the following Chemical Formula 1: 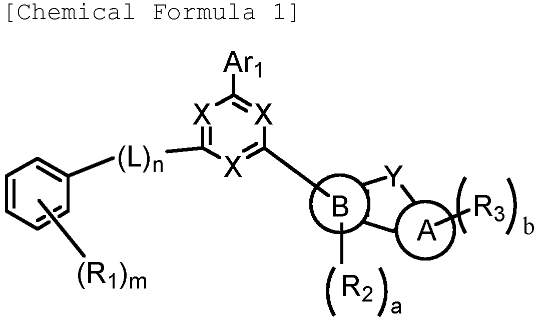
a plurality of X are the same or different from each other and are each independently CR₅ or N, with a proviso that at least two of the plurality of X are each N,
Ar₁ is selected from the group consisting of a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms,
Y is O, S, or NR₄,
Ring A and Ring B are the same as or different from each other, each independently being a C₅ to C₁₈ monocyclic or polycyclic hydrocarbon ring that may or may not include heteroatoms,
R₂ to R₃ are the same or different from each other and are each independently selected from the group consisting of a deuterium, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms, or combine with an adjacent group to form a fused ring;
R₄ to R₅ are same or different and are each independently selected from the group consisting of a hydrogen, a deuterium (D), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms;
L is selected from the group consisting of an arylene of C₆-C₁₈ and a heteroarylene of 5 to 18 nuclear atoms;
R₁ is selected from the group consisting of a C₆ to C₆₀ aryl group, a heteroaryl group having 5 to 60 nuclear atoms, and a C₆ to C₆₀ arylsilyl group;
m is an integer of 1 or greater, n is an integer of 0 to 3,
a is an integer of 0 to 2, b is an integer of 0 to 4,
the arylene and heteroarylene groups of L, the aryl, heteroaryl, and arylsilyl groups of R₁, and the alkyl, alkenyl, alkynyl, cycloalkyl, heterocycloalkyl, aryl, heteroaryl, alkyloxy, aryloxy, alkylsilyl, arylsilyl, alkylborone, arylboron, arylphosphine, arylphosphine oxide, and arylamine groups of R₂ to R₅ and Ar₁ are optionally each independently unsubstituted or substituted with at least one selected from the group consisting of a hydrogen, a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₁ to C₄₀ arylphosphine group, a C₆ to C₆₀ aryl phosphine oxide group, a C₆ to C₆₀ arylamine group, a C₅ to C₆₀ arylheteroarylamine group, and a heteroarylamine group having 5 to 60 nuclear atoms, and wherein when the substituent is present in a plural number, they are optionally the same or different from each other, and
provided that the total number of carbon atoms contained in Y-containing ring substituted or unsubstituted with the substituents comprises at least 16.

In addition, the present invention also provides an organic electroluminescent device including: an anode, a cathode, and one or more organic layers disposed between the anode and the cathode, wherein at least one of the one or more organic layers includes the compound represented by Chemical Formula 1.

In such a case, the organic layer including the compound represented by Chemical Formula 1 may be selected from a light emitting layer, a light emitting auxiliary layer, a hole injection layer, a hole transport layer, an electron injection layer, a lifespan improvement layer, an electron transport layer, and an auxiliary electron transport layer. In such a case, the compound represented by Chemical Formula 1 may be included as a material for at least one of a phosphorescent host material of a light emitting layer, an electron transport layer and an auxiliary electron transport layer.

### EFFECTS OF THE INVENTION

According to an embodiment of the present invention, a compound represented by Chemical Formula 1 has excellent characteristics such as an electron transport ability, luminescence ability, heat resistance, and the like, and thereby is applicable as an organic layer material of an organic electroluminescent device.

In particular, when the compound represented by Chemical Formula 1 of the present invention is used as a phosphorescent host, an electron transport layer material or an auxiliary electron transport layer material, it may exhibit high thermal stability, low driving voltage, rapid mobility, high current efficiency and long lifespan characteristics compared to conventional host materials or electron transport materials.

Accordingly, the organic electroluminescent device including the compound of Chemical Formula 1 may be significantly improved in aspects such as excellent light emitting performance, low driving voltage, long lifespan, and high efficiency, and thus may be effectively applied to full color display panels and the like.

Effects according to the present invention are not limited by the description exemplified above, and more diverse effects are included in the present specification.

### DETAILED DESCRIPTION OF THE INVENTION

Hereinafter, the present invention will be described in detail.

### <Novel organic compound>

The present invention provides a novel aryl compound exhibiting excellent thermal stability, carrier transport capability, and luminescence properties.

Specifically, the novel organic compound according to the present invention has a basic skeleton structure in which a plurality of aryl groups, and a ring compound having a non-covalent electron pair (e.g., a lone pair) such as a dibenzo/carbazole-based moiety, are bonded to an electron withdrawing group (EWG) having excellent electron transport ability.

The compound of chemical formula 1 having the structure described above structurally increases the electron density of EWG having electron transport ability by centering on an azine group (e.g., triazine, pyrimidine etc), which is a type of EWG group, and substituting a ring compound having a non-covalent electron pair on one side of the azine group and substituting a plurality of aryl groups on the other side. Accordingly, the electron transport ability is further improved compared to existing known material structures, resulting in an effect of increased efficiency. Further, the compound has a rigid chemical structure due to the ring compound such as dibenzo-based or carbazole-based ring compound bonded to the EWG group, and thus is excellent in terms of high glass transition temperature (Tg) and thermal stability.

Moreover, the compound represented by Chemical Formula 1 forms polycyclic structures by fusing an aryl group with a heterocyclic compound bound to the EWG. This design improves molecular electrical stability, leading to extended device lifespan compared to prior technologies. Direct binding of electron-donating heterocyclic compounds to the EWG typically concentrates HOMO and LUMO orbitals on the heterocyclic structure, increasing susceptibility to electrical damage and reducing device longevity. To solve this issue, the present invention incorporates fused polycyclic structures (e.g., Y-containing rings) with electron-donating heterocycles such as dibenzo moieties, significantly enhancing molecular stability. Furthermore, strong electron-withdrawing groups such as triazine or pyrimidine are introduced to improve electron mobility, thus providing physicochemical properties more suitable for electron injection and transport.

Additionally, the compound represented by Chemical Formula 1 of the present invention has a higher triplet energy level than the emission layer, thus preventing excitons generated in the emission layer from diffusing to adjacent electron transport layers or hole transport layers. This results in an increased number of excitons contributing to luminescence within the emission layer, improving device luminescence efficiency, durability, and stability, thereby extending device lifespan. Most of the developed materials exhibit physical properties that enable low-voltage operation, further improving device longevity.

As described above, the compound represented by Chemical Formula 1 of the present invention may be applicable as an organic layer material of an organic electroluminescent device, preferably a light-emitting layer material (a blue, a green and/or a red phosphorescent host material), an electron transport/injection layer material, a hole transport/injection layer material, a light-emitting auxiliary layer material, an auxiliary electron transport layer, and/or a lifespan enhancement layer material. In addition, the organic electroluminescent device including the compound of the above chemical formula may be greatly improved in terms of the performance and lifespan characteristics, and the performance a full color organic electroluminescent panel to which the organic electroluminescent device is applied may also be maximized.

According to the present invention, the compound represented by Chemical Formula 1 is bonded to a ring compound condensed to an electron withdrawing group (EWG) having excellent electron transport ability and a plurality of aryl groups to form a basic skeleton structure.

According to the present invention, the compound represented by Chemical Formula 1 has a basic skeleton structure in which a ring compound (e.g., Y-containing ring) and a plurality of aryl groups (e.g., R₁-containing ring) are bonded to an electron withdrawing group (EWG) having excellent electron transport ability, such as a nitrogen-containing heterocycle as a center.

In Chemical Formula 1, the nitrogen-containing heterocycle (e.g., a ring containing X) may be a monocyclic heteroaryl group (e.g., azine) containing at least two nitrogen atoms. For an example of the nitrogen-containing heteroaromatic ring (e.g., the ring containing X), a plurality of X may be the same as or different from each other, and may each independently be N or CR₅, provided that at least two of the plurality of X are N. For a specific example, a plurality of X includes two to three nitrogen atoms (N), preferably three nitrogen atoms (N). As such, since the heterocycle containing two to three nitrogen atoms (N) is included, more excellent electron absorption characteristics may be exhibited, which is advantageous for electron injection and transport.

Herein, R₅ in the nitrogen-containing heteroaromatic ring (e.g., X-containing ring) may be plural in number, and in such a case, the plurality of R5 may be the same as or different from each other and may each independently be selected from: hydrogen, deuterium (D), halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms. Specifically, R₅ may preferably be selected from: hydrogen, deuterium, a cyano group, a C₁ to C₄₀ alkyl group, a C₆ to C₆₀ aryl group, and a heteroaryl group having 5 to 60 nuclear atoms.

In an embodiment of the present invention, the nitrogen-containing heterocycle (e.g., X-containing ring) may be embodied into any one of Chemical Formulas 2 to Chemical Formula 5 below. 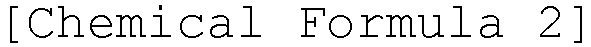 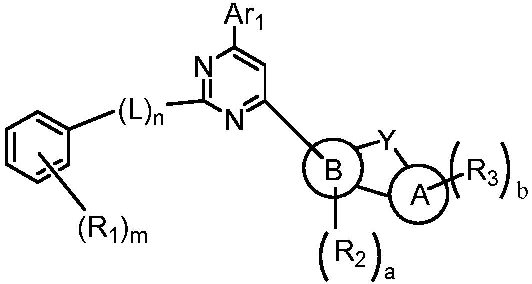 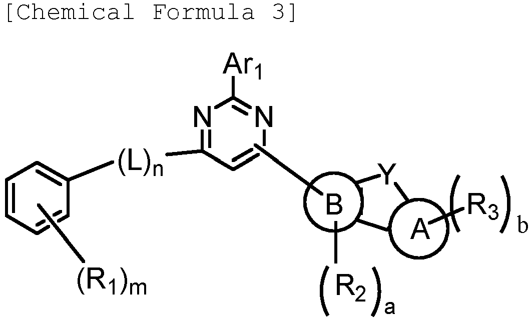 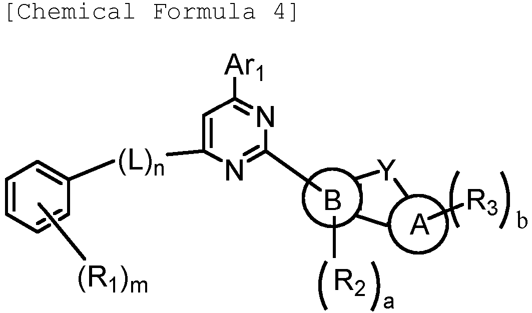 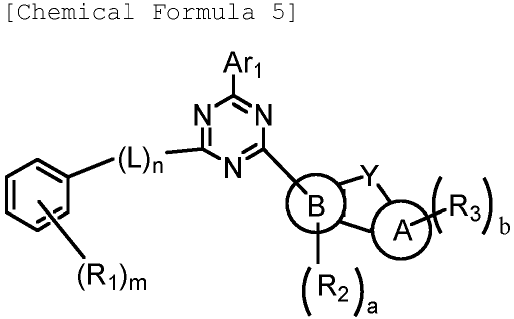 wherein the formulas,
A, B, Y, L, R₁~R₃, Ar1, a, b, m and n are each as defined in Chemical Formula 1.

The nitrogen-containing heterocycle (e.g., X-containing ring) according to the present invention may each be substituted with various substituents, e.g., Y-containing ring and Ar₁. Such Ar₁ is selected from the group consisting of a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms. Specifically, Ar₁ may be selected from: a C₆ to C₆₀ aryl group, and a heteroaryl group having 5 to 60 nuclear atoms, more specifically, Ar₁ may be preferably selected from: a C₆ to C₁₈ aryl group, and a heteroaryl group having 5 to 18 nuclear atoms.

In an embodiment of the present invention, Ar₁ may be a substituent represented by the following structural formula: 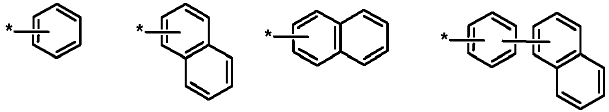 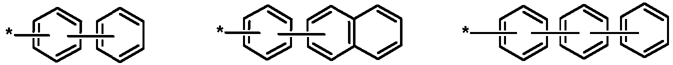 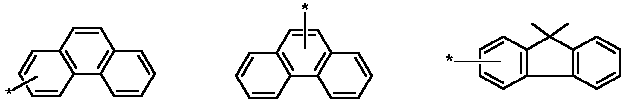 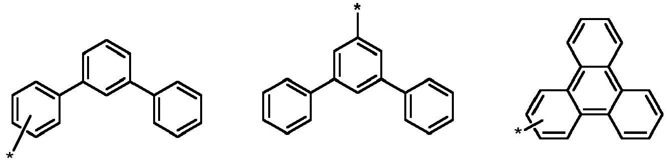 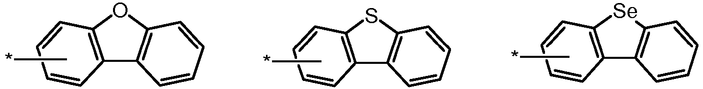 wherein the formulas,
* may indicate a site where a bond with Chemical Formula 1 is made.

The heterocyclic compound introduced into the nitrogen-containing heterocycle (X-containing ring) may be represented as a Y-containing ring in Chemical Formula 1, and specifically has a fused polycyclic heterocycle in which a typical aryl group is fused to a dibenzo/carbazole moiety.

In an embodiment of the present invention, Y is O, S, or NR₄.

Herein, R₄ may be a hydrogen, a deuterium (D), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms. Specifically, R₄ may be preferably selected from: a C₆ to C₃₀ aryl group, and a heteroaryl group having 5 to 30 nuclear atoms.

Ring A and Ring B may be the same as or different from each other, each independently a C₅ to C₁₈ monocyclic or polycyclic hydrocarbon ring that may or may not include heteroatoms, and provided that at least one of rings A and B is a polycyclic hydrocarbon ring of C₁₀-C₁₈. The ring A and ring B may be condensed or fused monocyclic or polycyclic hydrocarbon rings or nitrogen-containing rings known in the art, and may be, for example, monocyclic or polycyclic alicyclic rings, monocyclic or polycyclic heteroalicyclic rings, monocyclic or polycyclic aromatic rings, or monocyclic or polycyclic heteroaromatic rings. Preferably, they may be a polycyclic aromatic ring having 10 to 18 carbon atoms, or a polycyclic heteroaromatic ring having 10 to 12 nuclear atoms containing at least one heteroatom. Here, the heteroatom may be selected from the group consisting of N, O, and S.

R₂ to R₃ may be the same or different from each other and are each independently selected from the group consisting of a deuterium, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms, or combine with an adjacent group to form a monocyclic or polycyclic fused ring in the art. Preferably, R₂ to R₃ may be each independently selected from: a C₆ to C₆₀ aryl group, and a heteroaryl group having 5 to 60 nuclear atoms.
a is an integer of 0 to 2, b is an integer of 0 to 4. If a equals 0, the ring A does not have any substituent, with the exception of hydrogen; if a is 1 or 2, the ring A may have a substituent other than hydrogen. Similarly, if b equals 0, hydrogen exists on the ring B; if b is 1 or 2, the ring B may have the aforementioned substituents.

In the present invention, provided that the Y-containing rings may or may not have a substituent described hereinafter, with the total number of carbons in the substituted or unsubstituted Y-containing ring being at least 16, preferably the substituted or unsubstituted Y-containing ring includes 16 to 24 carbon atoms.

In an embodiment of the present invention, the Y-containing ring may be more specifically embodied as any one selected from the following structural formulas. However, it is not limited thereto.

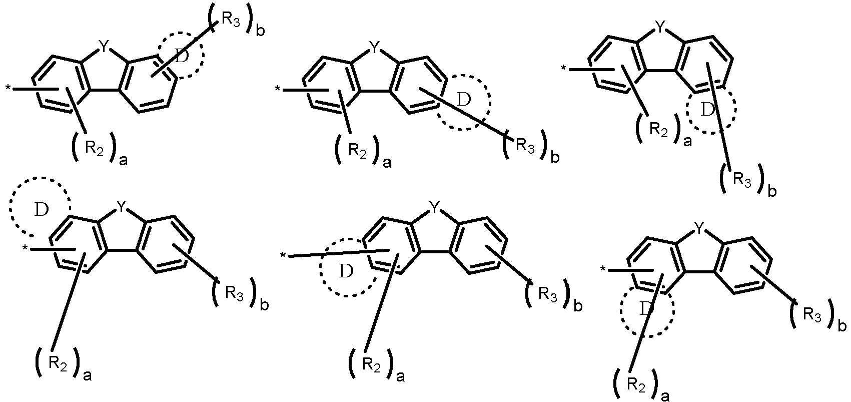

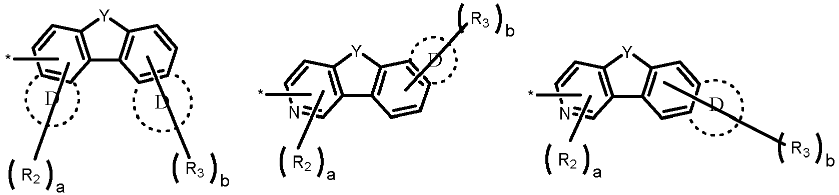

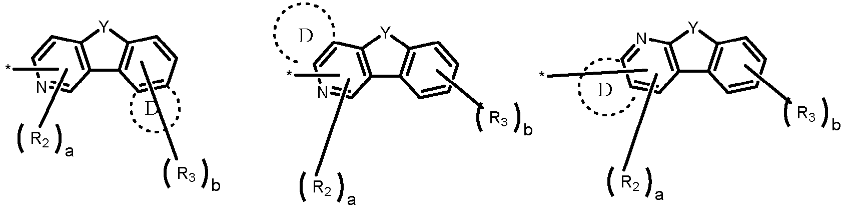

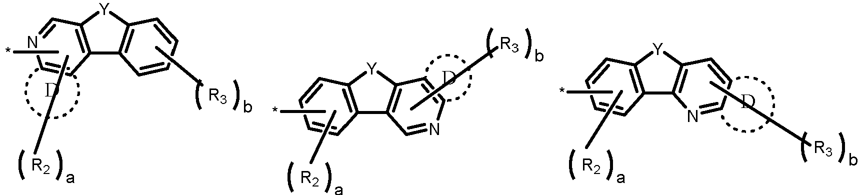

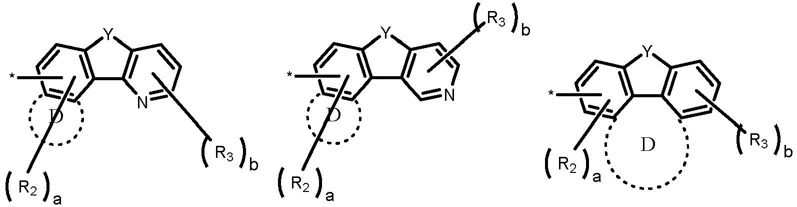

In the above structural formulas,
* may indicate a site where a bond to Chemical Formula 1 is made,
Ring D may be the same as or different from each other, each independently a monocyclic or polycyclic hydrocarbon ring group containing or not containing heteroatoms, and
Y, R₂, R₃, a and b are each as defined in Chemical Formula 1.

For one specific example, the Y-containing ring may be any one selected from the following structural formulas.

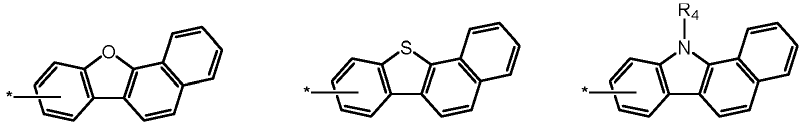

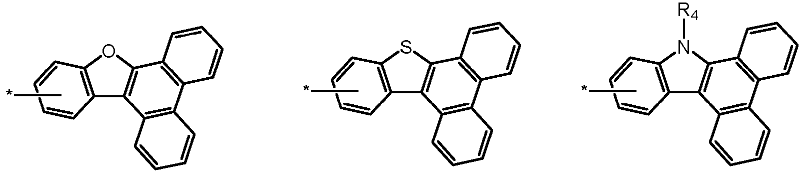

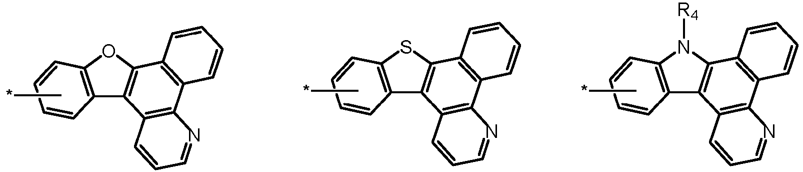

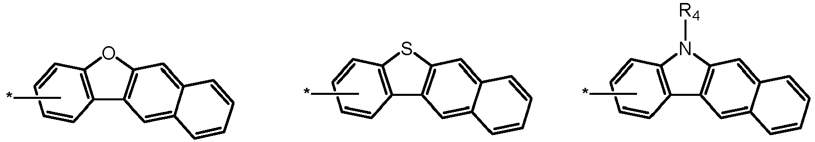

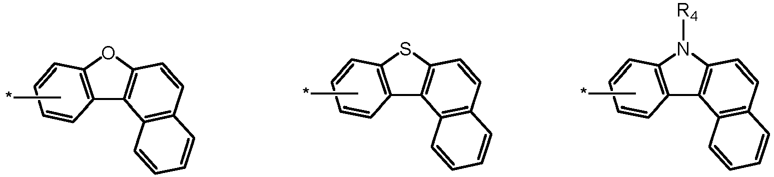

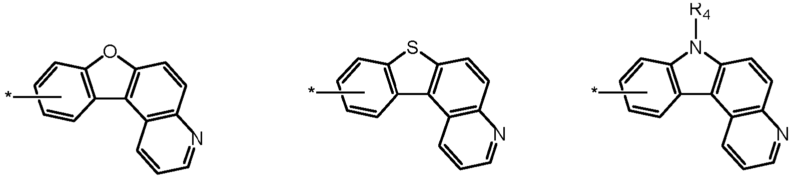

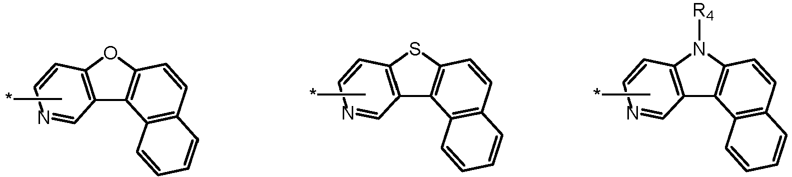

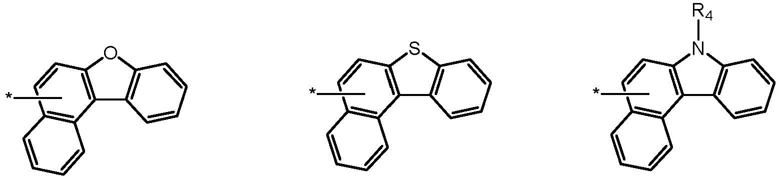

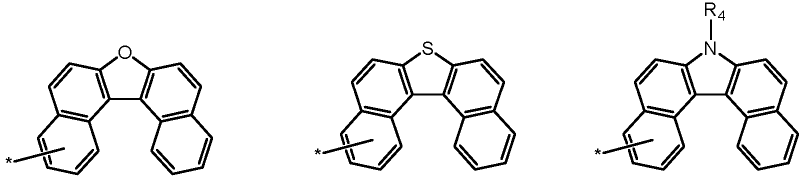

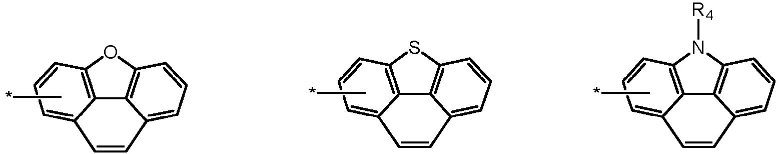

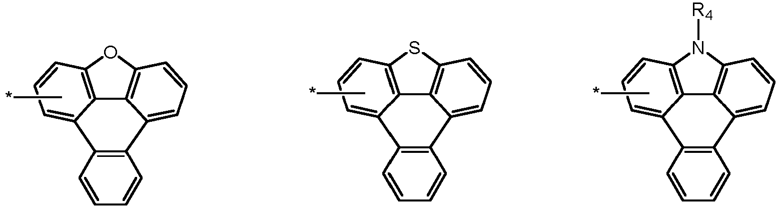

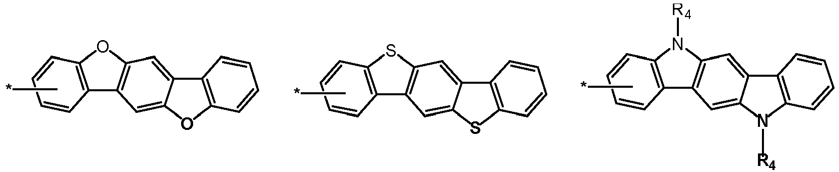

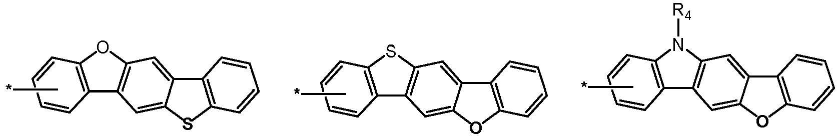

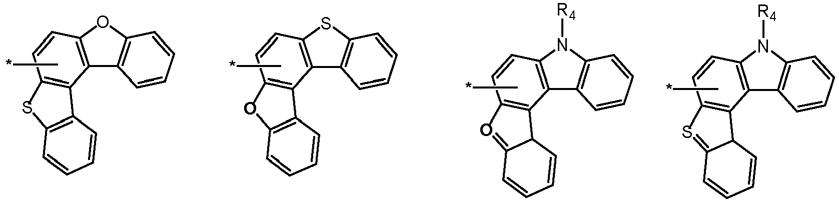

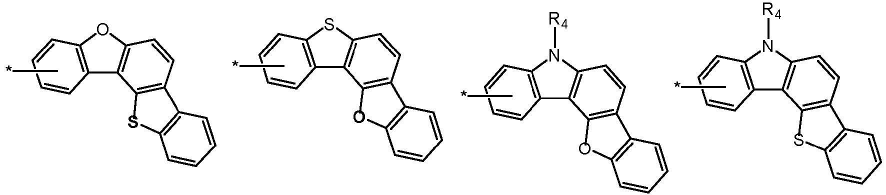

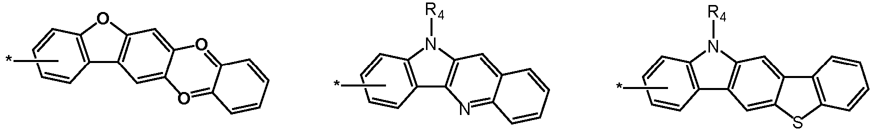

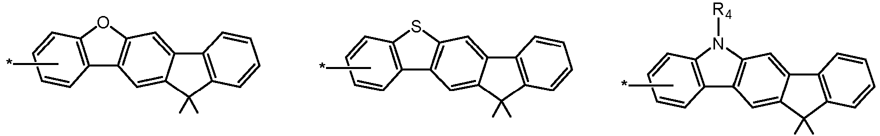

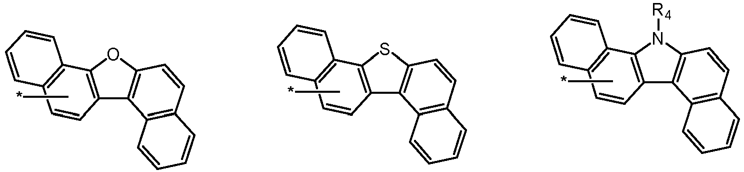

In the above structural formulas,
R₄ is as defined in Claim 1.

In addition, although not shown in the above structural formulas, at least one substituent known in the art (e.g., the same as the definition of R₂ to R₃) may be substituted.

The nitrogen-containing heterocycle (X-containing ring) according to the present invention may be bonded to a phenyl group substituted with a plurality of aryl groups (R₁), which are connected via a direct bond (n=0) or a separate linker (L). As such, when a separate linker (L) is present between the nitrogen-containing heteroaromatic ring and the phenyl group substituted with a plurality of the aryl groups, a HOMO region may be expanded to give a benefit to a HOMO-LUMO distribution, and charge transfer efficiency may be increased through an appropriate overlap of HOMO-LUMO.

The linker (e.g., L) are not particularly limited, and may be a common divalent group linker known in the art. Specifically, L may be selected from: a C₆ to C₁₈ arylene group and a heteroarylene group having 5 to 18 nuclear atoms. More specifically, L may be selected from: a C₆ to C₁₂ arylene group and a heteroarylene group having 5 to 12 nuclear atoms.

Specific examples of the arylene group and the heteroarylene group may include, for example, a phenylene group, a biphenylene group, a naphthylene group, an anthracenylene group, an indenylene group, a pyrantrenylene group, a carbazolylene group, a thiophenylene group, an indolylene group, a purinylene group, a quinolinylene group, a pyrrolylene group, an imidazolylene group, an oxazolylene group, a thiazolylene group, a triazolylene group, a pyridinylene group, and a pyrimidinylene group. And specific examples of the heteroarylene group may include, for examples, a dibenzofuran-based moiety, a dibenzothiophene-based moiety, and/or a dibenzoselenophenebased moiety. More specifically, L may be preferably selected from: a phenylene group, and a biphenylene group.

The compound of the Chemical Formula 1 according to the present invention includes a plurality of aryl groups/heteroaryl groups (e.g., R₁-containing rings) bonded to one side of a nitrogen-containing heterocycle (e.g., X containing ring).

Specifically, R₁ may be selected from the group consisting of a C₆ to C₆₀ aryl group, a heteroaryl group having a nuclear atom number of 5 to 60 and a C₆ to C₆₀ arylsilyl group. The number (e.g., m) of aryl groups/heteroaryl groups/arylsilyl groups may be an integer of 1 or more, and may be preferably 1 to 5. Here, when m is 2 to 5, a plurality of R1s may be the same or different.

In an embodiment of the present invention, R₁ may be selected from the group consisting of a C₆ to C₃₀ aryl group, a heteroaryl group having a nuclear atom number of 5 to 30, and a C₆ to C₆₀ arylsilyl group, m may be an integer of 2 to 5, and the total number of carbon atoms contained in (R₁)ₘ substituted or unsubstituted with a substituent described below may include at least 18. More specifically, R₁ may be a C₆ to C₁₈ aryl group, or a C₆ to C₂₄ arylsilyl group, m is an integer of 3 to 5, and the total number of carbon atoms contained in (R₁)ₘ substituted or unsubstituted with a substituent may include 18 to 36 carbon atoms. In the above Chemical Formula 1, the arylene and heteroarylene groups of L; the aryl, heteroaryl, arylsilyl groups of R₁; the alkyl, alkenyl, alkynyl, cycloalkyl, heterocycloalkyl, aryl, heteroaryl, alkyloxy, aryloxy, alkylsilyl, arylsilyl, alkylboron, arylboron, arylphosphine, monoarylphosphinyl, diarylphosphinyl, arylamine, arylheteroarylamine, and heteroarylamine groups of R₂ to R₅ and Ar₁ are optionally each independently unsubstituted or substituted with at least one selected from the group consisting of a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₁-C₄₀ arylphosphine group, a C₆-C₆₀ aryl phosphine oxide group, a C₆-C₆₀ arylamine group, a C₅-C₆₀ 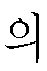 arylheteroarylamine group, and a heteroarylamine group having 5 to 60 nuclear atoms, and provided that when the substituent is present in a plural number, they are optionally the same or different from each other.

In an embodiment of the present invention, the compound represented by Chemical Formula 1 may be represented by any one of the following Chemical Formulas 6 to 13 according to the type of the linker (L) and the cyclic compound (Y-containing ring) having a lone pair of electrons, specifically a dibenzo-based/carbazole-based moieties (e.g., Ar₁). However, it is not limited thereto.

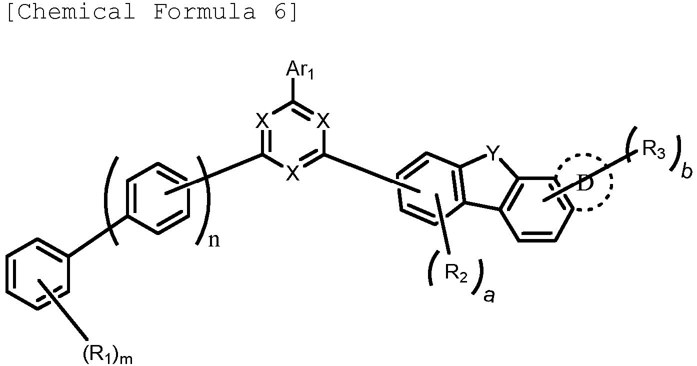

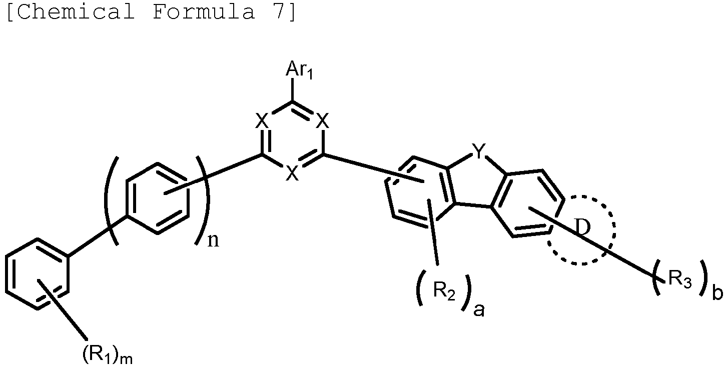

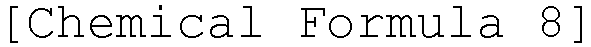

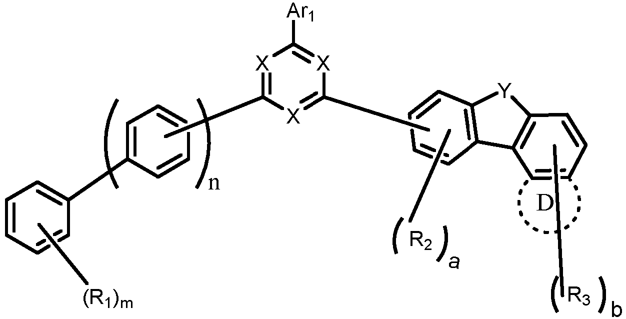

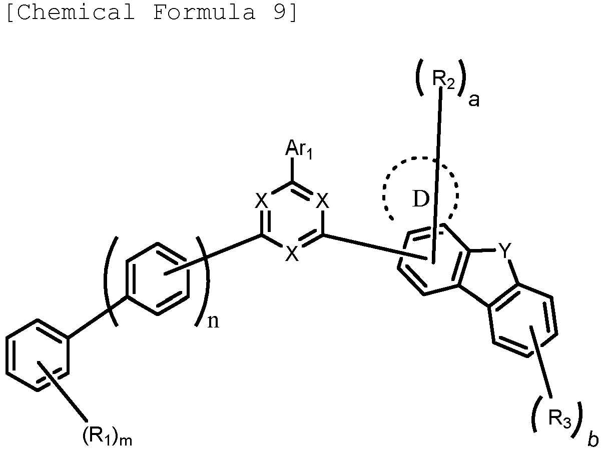

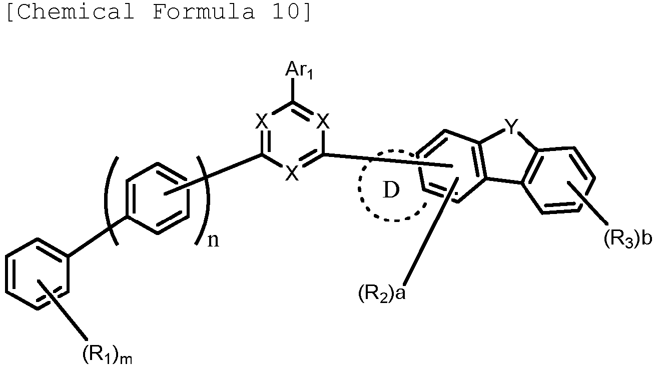

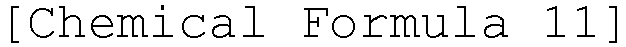

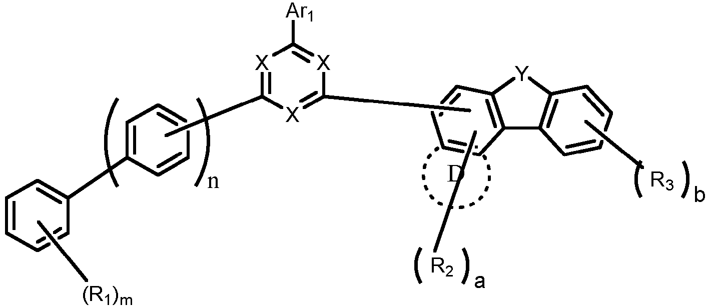

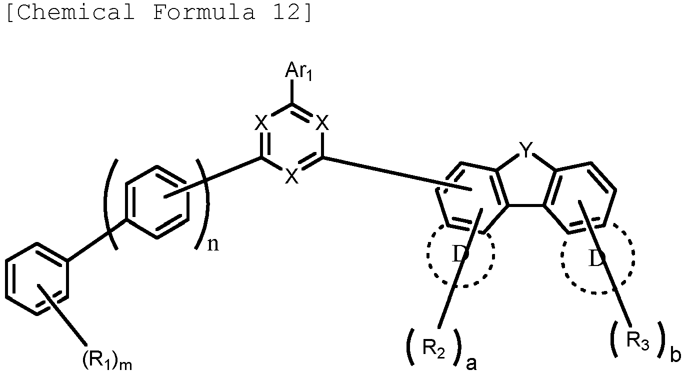

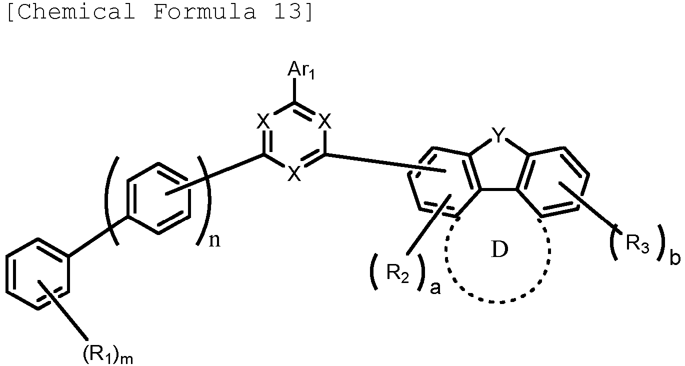

In above Chemical Formulas,
Ring D is a monocyclic or polycyclic hydrocarbon ring group that may or may not include heteroatoms,
X, Y, R₁~R₃, Ar₁, a, b, m and n are each as defined in Chemical Formula 1

The compounds represented by Formulas 9 to 11 described above have a structure in which a polycyclic fused ring (e.g., d) is disposed adjacent to an azine group, and the compounds represented by Formulas 6 to 8 are disposed relatively far from the azine group. In this way, when the condensate ring is not adjacent to the azine group, the LUMO region is mainly distributed in the azine group and does not evenly spread to the condensate ring, so it shows fast electron transport ability. In addition, since the conjugation length is longer than the non-condensed compound, the molecular stability can be increased to improve the lifetime of the device.

In another embodiment of the present invention, the compound represented by Chemical Formula 1 may be represented by any one of the following Chemical Formulas 14 to 25 according to the number of aryl groups (R₁) introduced into the molecule and their bonding positions. However, it is not limited thereto.

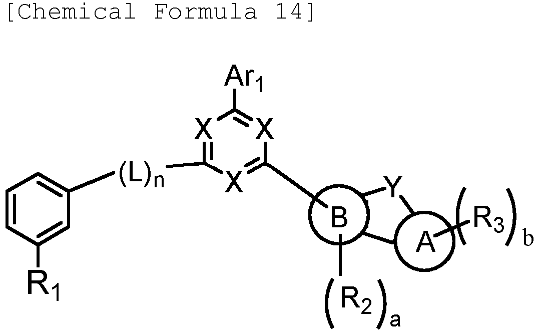

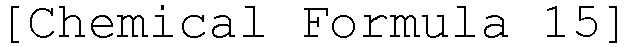

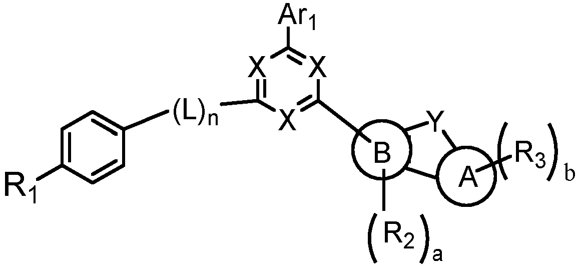

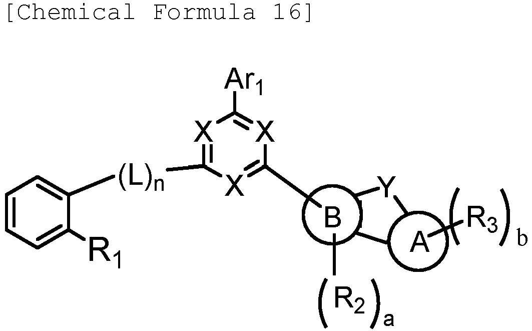

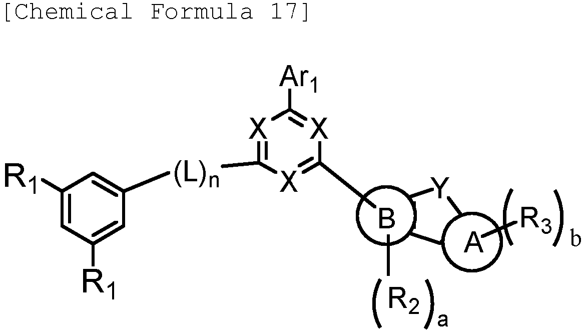

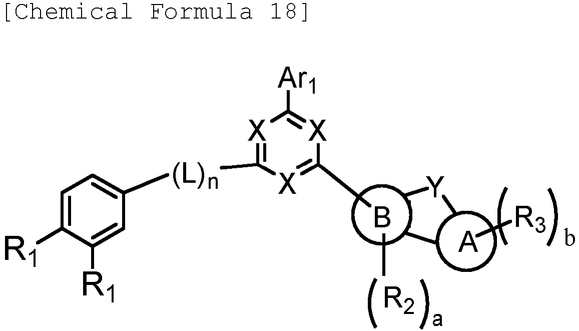

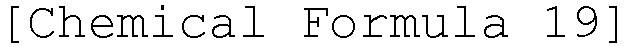

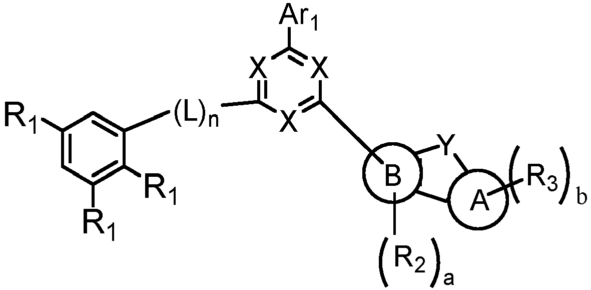

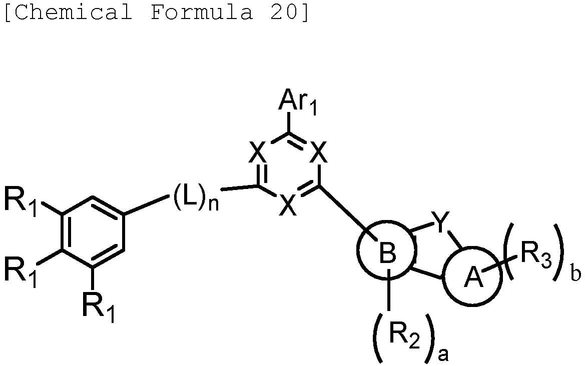

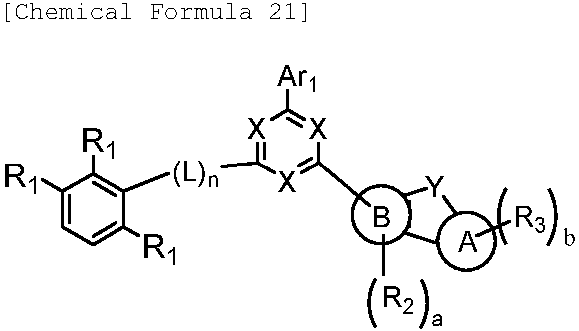

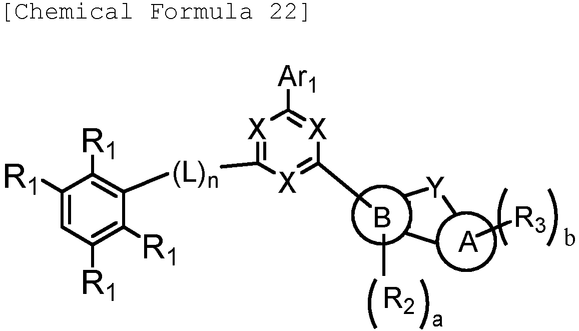

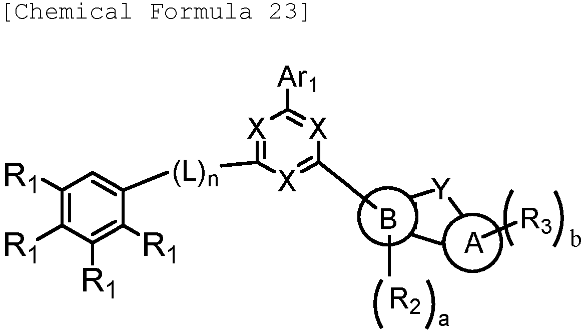

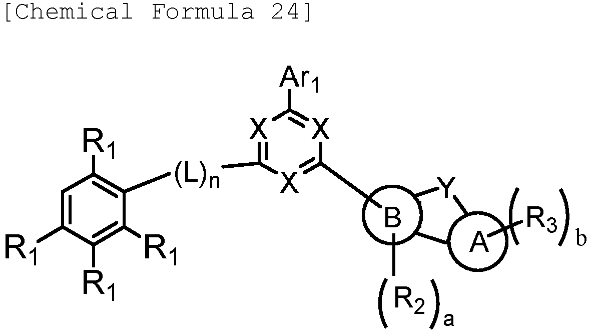

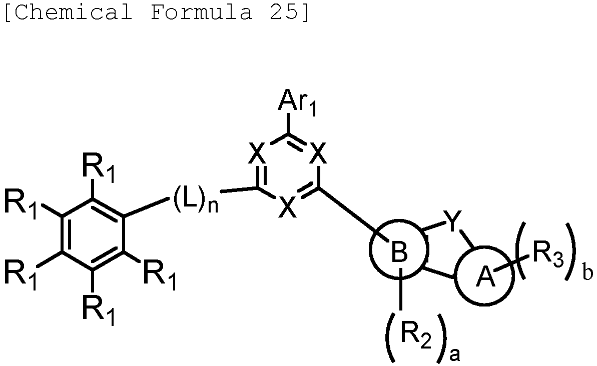

In above Chemical Formulas,
X, Y, A, B, Ar₁, L, R₁~R₃, a, b, and n are each as defined in Chemical Formula 1.

The compound represented by Chemical Formula 1 of the present invention described above may be further embodied as compounds represented by the following compounds represented by, for example, 1 to 108. However, the compound represented by Chemical Formula 1 of the present invention is not limited to those exemplified below.

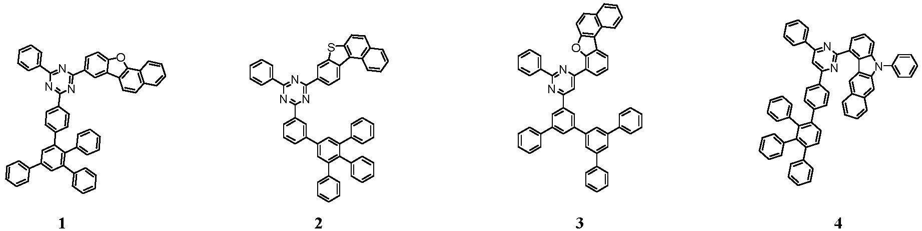

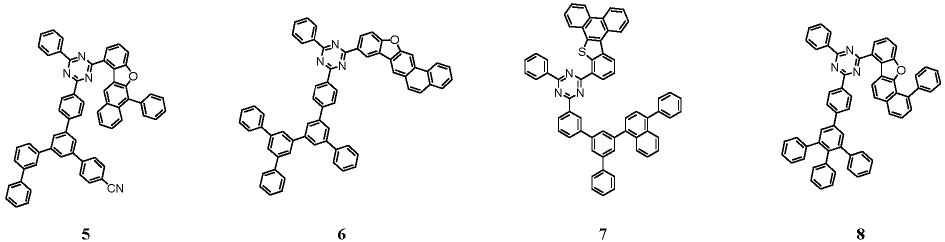

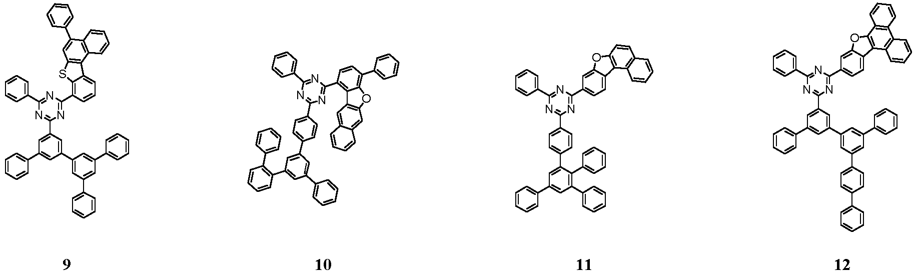

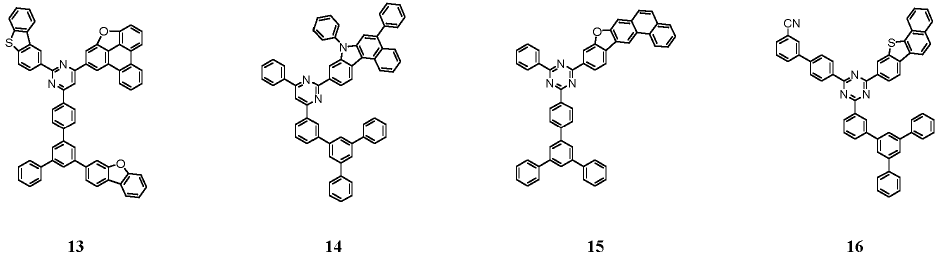

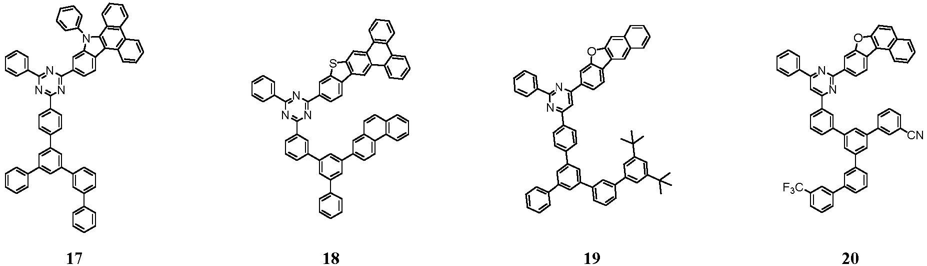

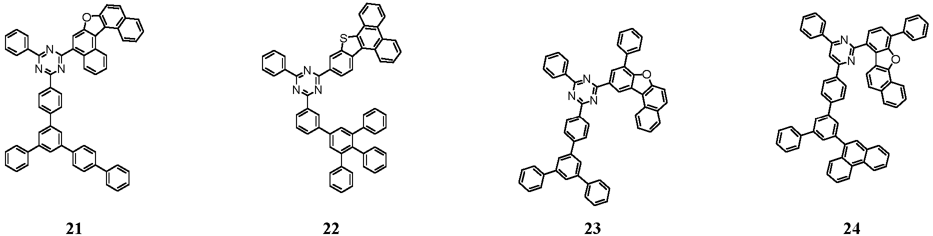

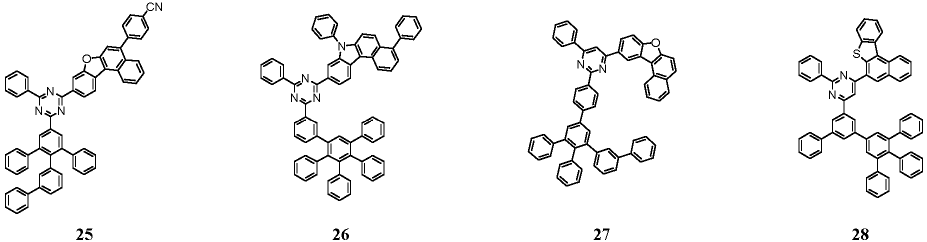

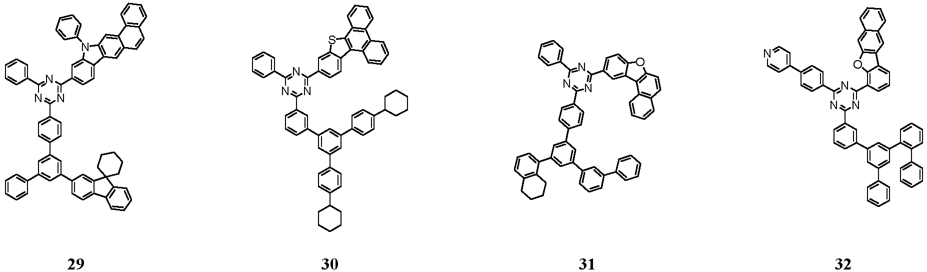

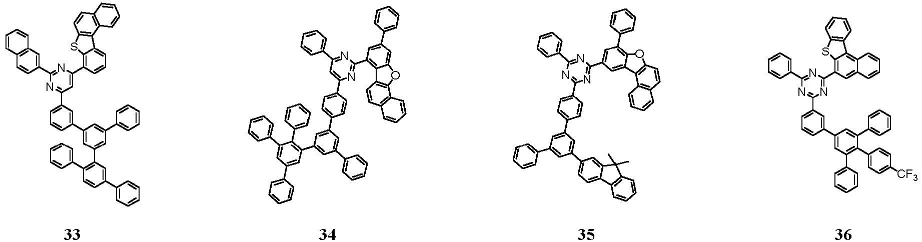

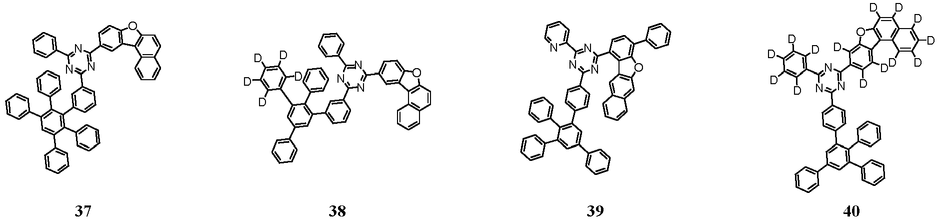

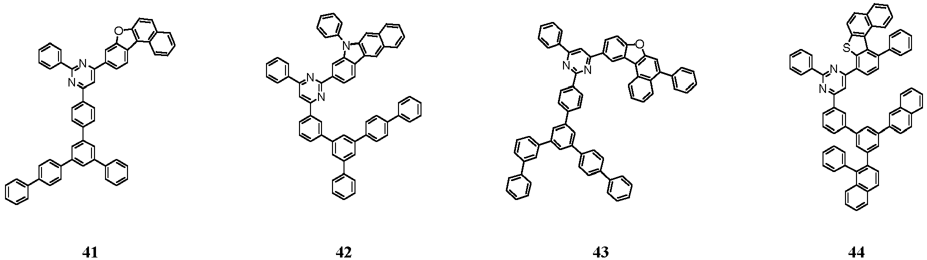

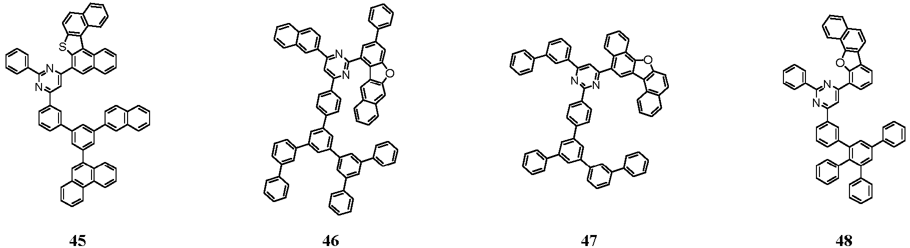

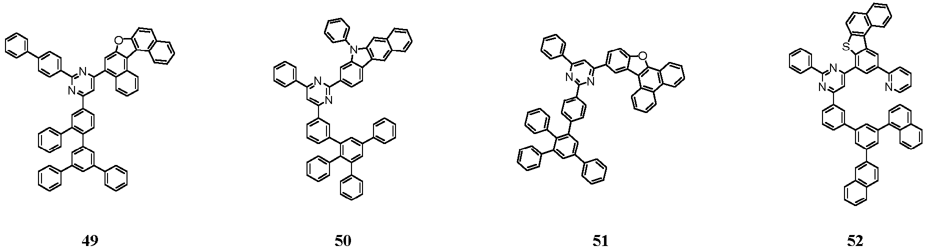

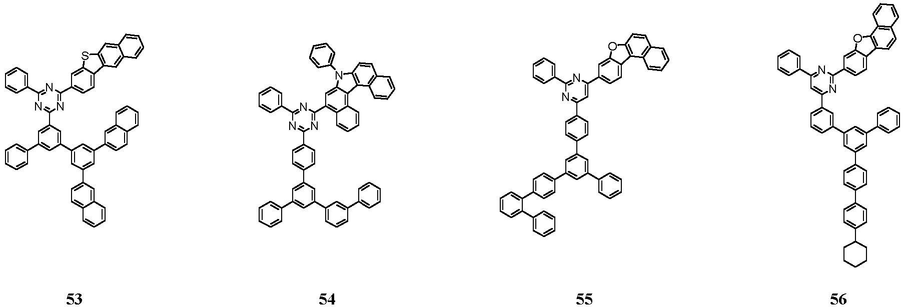

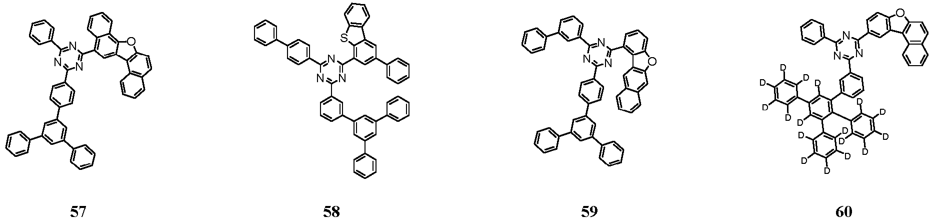

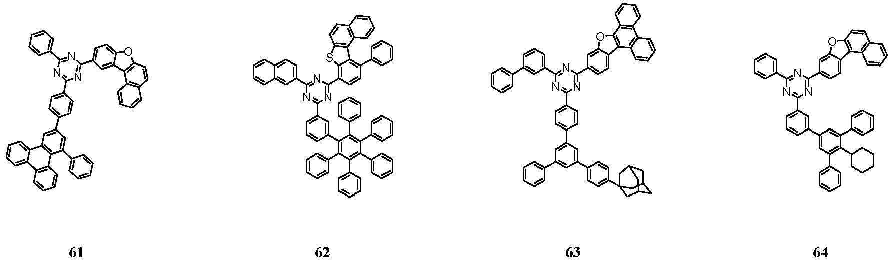

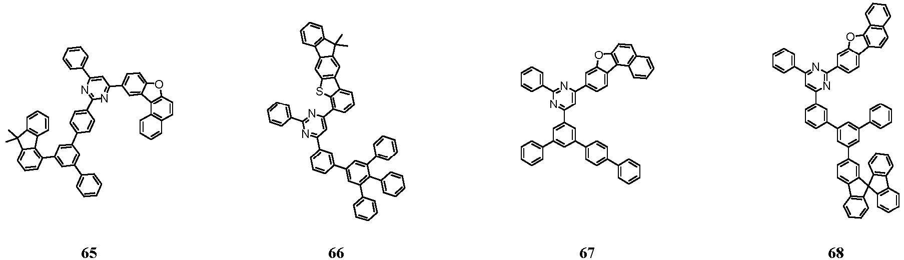

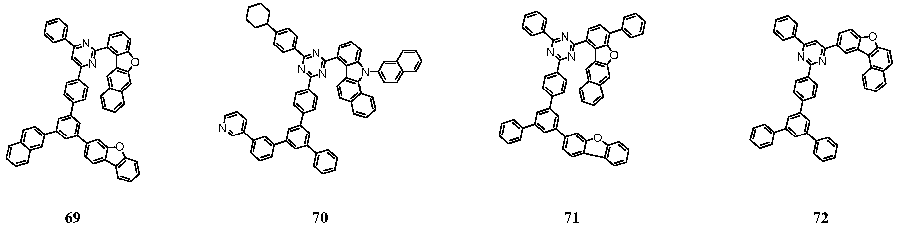

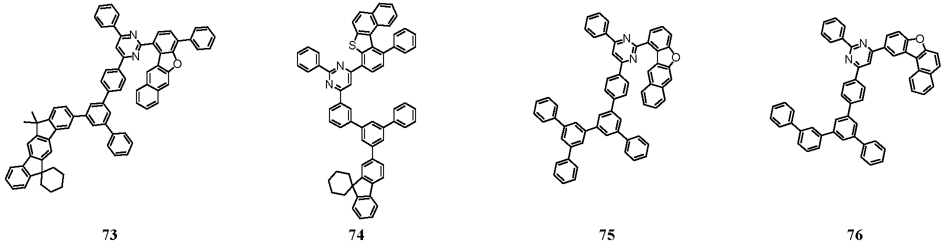

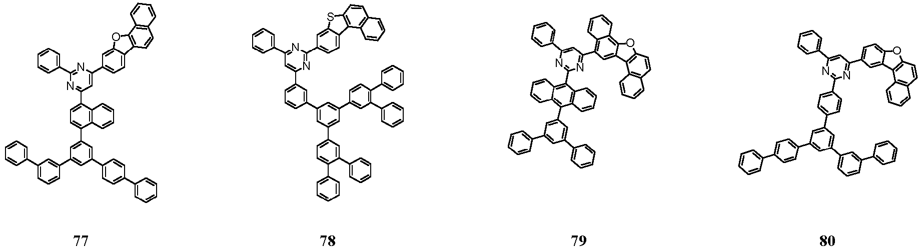

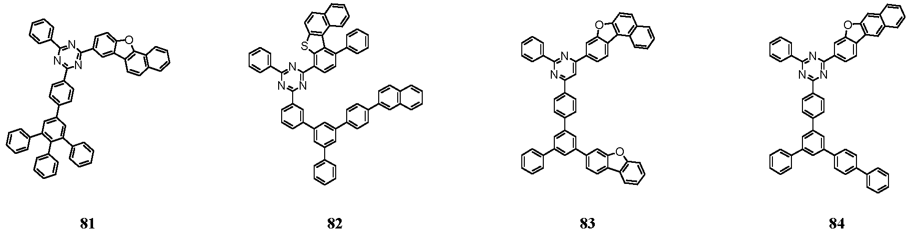

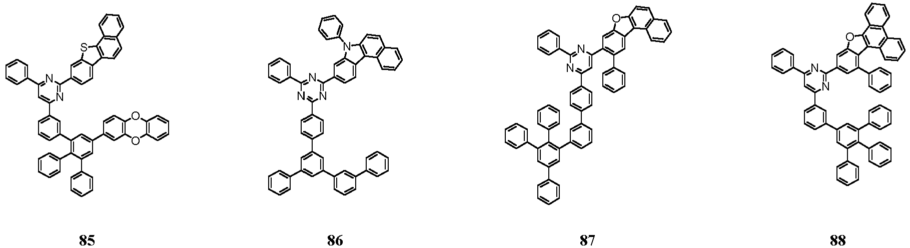

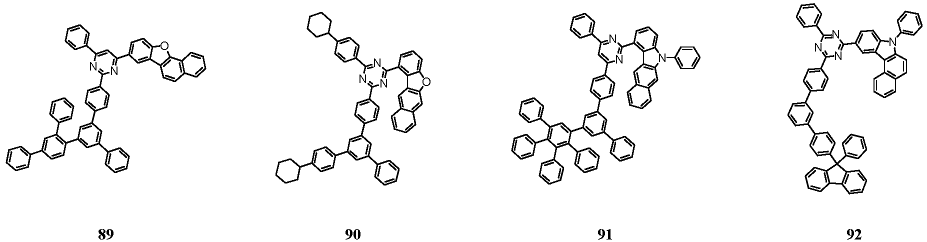

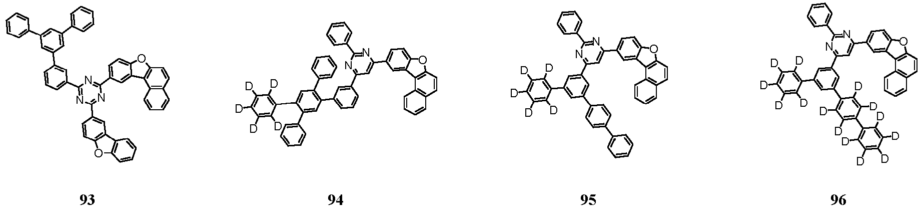

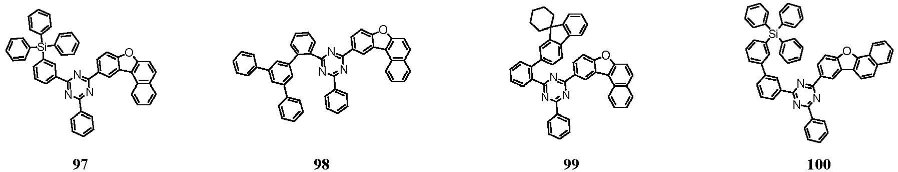

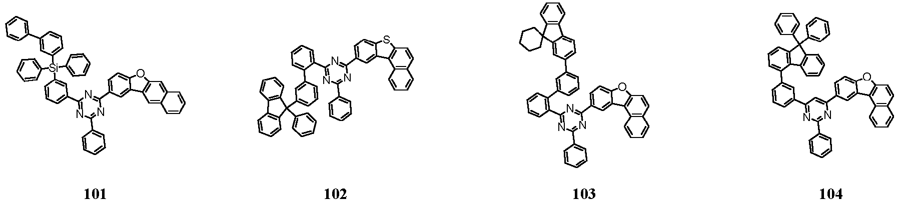

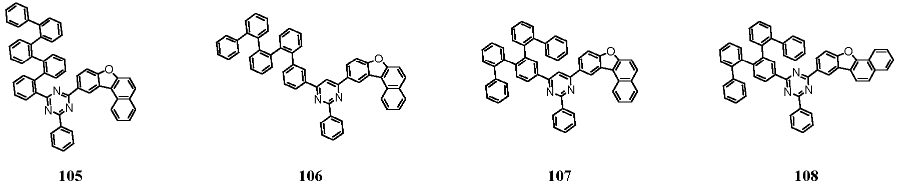

As used herein, "alkyl" refers to a monovalent substituent derived from a linear or branched chain saturated hydrocarbon having 1 to 40 carbon atoms. Examples of such alkyl may include, but not limited to, methyl, ethyl, propyl, isobutyl, sec-butyl, pentyl, iso-amyl, hexyl or the like.

As used herein, "alkenyl" refers to a monovalent substituent derived from a C₂ to C₄₀ linear or branched chain unsaturated hydrocarbon, having at least one carbon-carbon double bond. Examples of such alkenyl may include, but not limited to, vinyl, allyl, isopropenyl, 2-butenyl or the like.

As used herein, "alkynyl" refers to a monovalent substituent derived from a C₂ to C₄₀ linear or branched chain unsaturated hydrocarbon, having at least one carbon-carbon triple bond. Examples of such alkynyl may include, but not limited to, ethynyl, 2-propynyl or the like.

As used herein, "aryl" refers to a monovalent substituent derived from a C₆ to C₄₀ aromatic hydrocarbon having a structure with a single ring or two or more rings combined with each other. In addition, a form in which two or more rings are pendant (e.g., simply attached) to or fused with each other may also be included. Examples of such aryl may include, but not limited to, phenyl, naphthyl, phenanthryl, anthryl or the like.

As used herein, "heteroaryl" refers to a monovalent substituent derived from a monoheterocyclic or polyheterocyclic aromatic hydrocarbon having 5 to 40 nuclear atoms. In such an embodiment, one or more carbons in the ring, preferably one to three carbons, are substituted with a heteroatom such as N, O, S or Se. In addition, a form in which two or more rings are pendant to or fused with each other may be included and a form fused with an aryl group may be included. Examples of such heteroaryl may include, but not limited to, a 6-membered monocyclic ring such as pyridyl, pyrazinyl, pyrimidinyl, pyridazinyl and triazinyl; a polycyclic ring such as phenoxathienyl, indolizinyl, indolyl, purinyl, quinolyl, benzothiazole, and carbazolyl; 2-furanyl; N-imidazolyl; 2-isoxazolyl; 2-pyridinyl; 2-pyrimidinyl or the like.

As used herein, "aryloxy" is a monovalent substituent represented by RO-, where R refers to a C₅ to C₄₀ aryl. Examples of such aryloxy may include, but not limited to, phenyloxy, naphthyloxy, diphenyloxy or the like.

As used herein, "alkyloxy" refers to a monovalent substituent represented by R'O-, where R' refers to a C₁ to C₄₀ alkyl. Such alkyloxy may include a linear, branched or cyclic structure. Examples of such alkyloxy may include, but not limited to, methoxy, ethoxy, n-propoxy, 1-propoxy, t-butoxy, n-butoxy, pentoxy or the like.

As used herein, "arylamine" refers to amine substituted with a C₆ to C₄₀ aryl.

As used herein, "cycloalkyl" refers to a monovalent substituent derived from a C₃ to C₄₀ monocyclic or polycyclic non-aromatic hydrocarbon. Examples of such cycloalkyl may include, but not limited to, cyclopropyl, cyclopentyl, cyclohexyl, norbornyl, adamantine or the like.

As used herein, "heterocycloalkyl" refers to a monovalent substituent derived from a non-aromatic hydrocarbon having 3 to 40 nuclear atoms, where one or more carbons in the ring, preferably one to three carbons, are substituted with a heteroatom such as N, O, S or Se. Examples of such heterocycloalkyl may include, but not limited to, morpholine, piperazine or the like.

As used herein, "alkylsilyl" refers to silyl substituted with a C₁ to C₄₀ alkyl, and "arylsilyl" refers to silyl substituted with a C₅ to C₄₀ aryl.

As used herein, the term "fused ring (e.g., condensed ring)" refers to a condensed aliphatic ring, a condensed aromatic ring, a condensed heteroaliphatic ring, a condensed heteroaromatic ring, or a combination thereof.

### <Electron transport layer material>

The present invention provides an electron transport layer including the compound represented by Chemical Formula 1.

The electron transport layer (ETL) serves to move electrons injected from a cathode to an adjacent layer, specifically a light emitting layer.

The compound represented by Chemical Formula 1 may be used alone as an electron transport layer (ETL) material, or may be used in combination with an electron transport layer material known in the art. It may preferably be used alone.

The electron transport layer material that may be used in combination with the compound of Chemical Formula 1 may include an electron transport material commonly known in the art. Nonlimiting examples of applicable electron transport materials may include oxazole-based compounds, isoxazole-based compounds, triazole-based compounds, isothiazole-based compounds, oxadiazole-based compounds, thiadiazole-based compounds, perylene-based compounds, aluminum complexes (e.g., tris(8-quinolinolato)-aluminium (Alq₃), BAlq, SAlq, Almq₃, gallium complexes (e.g., Gaq'2OPiv, Gaq'2OAc, 2(Gaq'2)), etc. These may be used alone or two or more types thereof may be used in combination.

In the present invention, when the compound of Chemical Formula 1 and the electron transport layer material are used in combination, a mixing ratio thereof is not particularly limited, and may be appropriately adjusted within a range known in the art.

### <Auxiliary electron transport layer material>

In addition, the present invention provides an auxiliary electron transport layer including the compound represented by Chemical Formula 1.

The auxiliary electron transport layer is disposed between the light emitting layer and the electron transport layer and serves to substantially prevent diffusion of excitons or holes generated in the light emitting layer into the electron transport layer.

The compound represented by Chemical Formula 1 may be used alone as an auxiliary electron transport layer material, or may be combined with an electron transport layer material known in the art. It may preferably be used alone.

The auxiliary electron transport layer material that may be used in combination with the compound of Chemical Formula 1 includes an electron transport material commonly known in the art. For example, the auxiliary electron transport layer may include an oxadiazole derivative, a triazole derivative, a phenanthroline derivative (e.g., BCP), a heterocyclic derivative containing nitrogen, and the like.

In the present invention, when the compound of Chemical Formula 1 and the auxiliary electron transport layer material are used in combination, a mixing ratio thereof is not particularly limited, and may be appropriately adjusted within a range known in the art.

### <Organic electroluminescent device>

Another aspect of the present invention is directed to an organic electroluminescent device ("organic EL element") including the compound represented by Chemical Formula 1.

More specifically, the organic EL element according to the present invention includes an anode (e.g., a positive electrode), a cathode (e.g., a negative electrode), and one or more organic layers disposed between the anode and the cathode, and at least one of the one or more organic layers includes the compound represented by Chemical Formula 1. In such an embodiment, the compound may be used alone or in combination of two or more kinds thereof.

The one or more organic layers may be any one or more of a hole injection layer, a hole transport layer, a light emitting layer, a light emitting auxiliary layer, a lifespan improvement layer, an electron transport layer, an auxiliary electron transport layer and an electron injection layer, and at least one of the organic layers may include the compound represented by Chemical Formula 1. Specifically, the organic layer including the compound represented by Chemical Formula 1 may preferably be a light emitting layer (more specifically, a phosphorescence emitting host material), an electron transport layer, and an auxiliary electron transport layer.

The light emitting layer of the organic EL element of the present invention includes a host material and a dopant material, and may include the compound of Chemical Formula 1 as a host material. In addition, the light emitting layer of the present invention may include, as a host, other compounds known in the art rather than or in addition to the compound of Chemical Formula 1.

When the compound represented by Chemical Formula 1 is included as a material for the light emitting layer of the organic EL element, preferably as a blue, green, or red phosphorescent host material, since a bonding force between holes and electrons in the light emitting layer is increased, the efficiency (luminescence efficiency and power efficiency), lifespan, luminance, driving voltage, and the like of the organic EL element may be improved. Specifically, the compound represented by Chemical Formula 1 may be preferably included in an organic EL element as a green and/or red phosphorescent host, a fluorescent host, or a dopant material. In particular, the compound represented by Chemical Formula 1 of the present invention may preferably be a green phosphorescent exciplex N-type host material for a high-efficiency light emitting layer.

A structure of the organic EL element of the present invention is not particularly limited, and may be, for example, a structure in which a substrate, an anode, a hole injection layer, a hole transport layer, a light emitting auxiliary layer, a light emitting layer, an electron transport layer, and a cathode are sequentially stacked. In such a case, at least one of the hole injection layer, the hole transport layer, the light emitting auxiliary layer, the light emitting layer, the electron transport layer and the electron injection layer may include the compound represented by Chemical Formula 1, and preferably, the light emitting layer, more preferably a phosphorescent host, may include the compound represented by Chemical Formula 1. Meanwhile, an electron injection layer may be additionally stacked on the electron transport layer.

The organic EL element of the present invention may have a structure in which an insulating layer or an adhesive layer is inserted at an interface between the electrode and the organic layer.

The organic EL element of the present invention may be prepared using materials and methods known in the art to form organic layers and electrodes, except that one or more layers of the aforementioned organic layers include the compound represented by Chemical Formula 1.

The organic layer may be formed by a vacuum deposition method or a solution coating method. Examples of the solution coating method may include, but not limited to, spin coating, dip coating, doctor blading, inkjet printing, thermal transfer or the like.

The substrate used in preparation of the organic EL element of the present invention is not particularly limited, and nonlimiting examples thereof may include silicon wafers, quartz, glass plates, metal plates, plastic films, sheets or the like.

In addition, an anode material may use any anode material known in the art without limitation. Examples of the anode material may include, but not limited to, a metal such as vanadium, chromium, copper, zinc, and gold or an alloy thereof; metal oxides such as zinc oxide, indium oxide, indium tin oxide (ITO), or indium zinc oxide (IZO); combination of oxide with metal such as ZnO:Al or SnO₂:Sb; conductive polymers such as polythiophene, poly(3-methylthiophene), poly [3,4-(ethylene-1,2-dioxy) thiophene] (PEDT), polypyrrole or polyaniline; carbon black or the like.

In addition, a cathode material may use any cathode material known in the art without limitation. Examples of the cathode material may include, but not limited to, a metal such as magnesium, calcium, sodium, potassium, titanium, indium, yttrium, lithium, gadolinium, aluminum, silver, tin, or lead or an alloy thereof; a multi-layered material such as LiF/Al or LiO₂/Al or the like.

In addition, materials for the hole injection layer, the hole transport layer, the electron injection layer, and the electron transport layer are not particularly limited, and conventional materials known in the art may be used without limitation.

Hereinafter, the present invention will be described in detail through examples. However, the following examples are only to illustrate the present invention, and the present invention is not limited by the following examples.

### [Preparation Example 1]

### <Step 1> Synthesis of 3'-chloro-5'-phenyl-1,1':2',1"-terphenyl

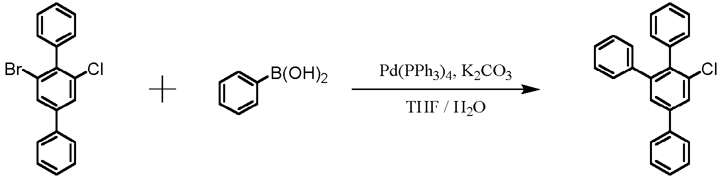

100 g (290.9 mmol) of 2'-bromo-6'-chloro-1,1':4',1"-terphenyl, 37.3 g (305.5 mmol) of phenylboronic acid, 10.1 g (8.7 mmol) of Pd(PPh₃)₄, and 120.6 g (872.98 mmol) of K₂CO₃ were added to 970 ml of THF and 240 ml of H₂O, and the mixture was heated under reflux with stirring for 6 hours. After the reaction was completed, the mixture was extracted with dichloromethane, and MgSO₄ was added to remove moisture, followed by filtering, and then the solvent of an organic layer was concentrated under reduced pressure, and the product was purified by column chromatography using dichloromethane and hexane. The purified product was solidified using methanol. The solid was filtered, washed with methanol, and dried in an oven to obtain the target compound, 3'-chloro-5'-phenyl-1,1':2',1"-terphenyl (75.6 g, yield 76.3%).
Mass : [(M+H)⁺] : 341

### <Step 2> Synthesis of Core 1

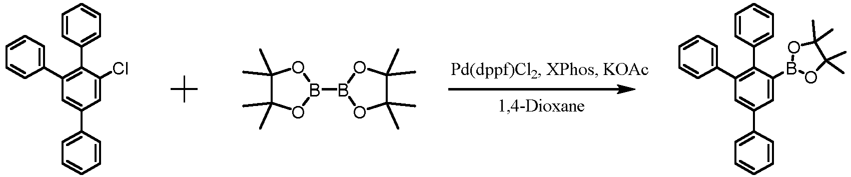

75.6 g (221.7 mmol) of 3'-chloro-5'-phenyl-1,1':2',1"-terphenyl, 73.2 g (288.3 mmol) of 4,4,4',4',5,5,5',5'-octamethyl-2,2'-bi(1,3,2-dioxaborolane), 5.4 g (6.7 mmol) of Pd(dppf)Cl₂, 65.3 g (665.4 mmol) of KOAc, 6.3 g (13.3 mmol) of Xphos were added to 750 ml of 1,4-Dioxane, and the mixture was heated under reflux for 12 hours. After the reaction was completed, the mixture was extracted with dichloromethane, and MgSO₄ was added to remove moisture, followed by filtering, and then the solvent of an organic layer was concentrated under reduced pressure, and the product was purified by column chromatography using dichloromethane and hexane. The purified product was solidified using methanol. The solid was filtered, washed with methanol, and dried in an oven to obtain the target compound, Core 1 (72.7g, yield 75.8%).
Mass : [(M+H)⁺] : 433

### [Preparation Example 2]

### <Step 1> Synthesis of 5'-chloro-5"-phenyl-1,1':3',1'':3'',1‴-quaterphenyl

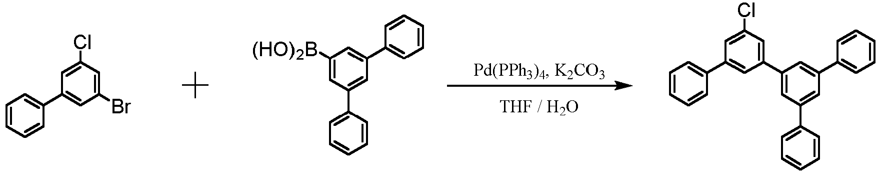

50 g (186.9 mmol) of 3-bromo-5-chloro-1,1'-biphenyl, 54.4 g (205.6 mmol) of [1,1':3',1"-terphenyl]-5'-ylboronic acid, 6.5 g(5.6 mmol) of Pd(PPh₃)₄, and 77.5 g(560.6 mmol) of K₂CO₃ were added to 700 ml of THF and 175 ml of H₂O, and the target compound, 5'-chloro-5''-phenyl-1,1':3',1'':3'',1‴-quaterphenyl (64.1 g, yield 82.3%) was obtained using the same manner as in Step 1 of Preparation Example 1.
Mass : [(M+H)⁺] : 417

### <Step 2> Synthesis of Core 2

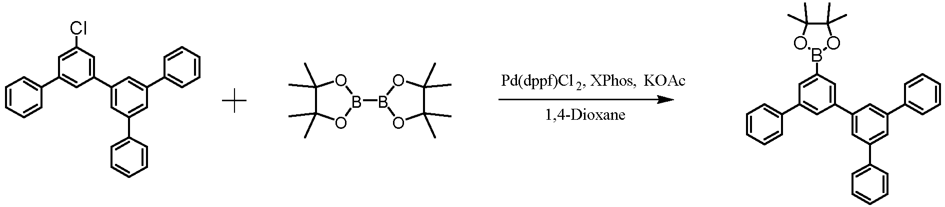

64.1 g (153.7 mmol) of 5'-chloro-5"-phenyl-1,1':3',1'':3'',1‴-quaterphenyl, 50.8 g (199.9 mmol) of 4,4,4',4',5,5,5',5'-octamethyl-2,2'-bi(1,3,2-dioxaborolane), 3.8 g(4.6 mmol) of Pd(dppf)Cl₂, 45.3 g (461.2 mmol) of KOAc, and 4.4 g (9.2 mmol) of Xphos were added to 600 ml of 1,4-Dioxanem and Core 2 (68.9 g, yield 88.1%) was obtained using the same manner as in Core 1 of Preparation Example 1.
Mass : [(M+H)⁺] : 508

### [Preparation Example 3]

### <Step 1> Synthesis of 4'-chloro-6'-phenyl-1,1':2',1":3",1‴-quaterphenyl

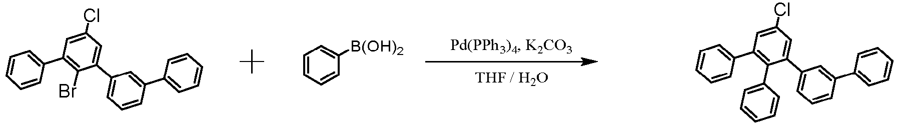

60 g(142.9 mmol) of 2'-bromo-5'-chloro-1,1':3',1'':3'',1'''-quaterphenyl, 19.2 g(157.2 mmol) of phenylboronic acid, 5.0 g(4.3 mmol) of Pd(PPh₃)₄, 59.3 g(428.8 mmol) of K₂CO₃ were added to 600 ml of THF, 150 ml of H₂O, and the target compound, 4'-chloro-6'-phenyl-1,1':2',1'':3'',1‴-quaterphenyl (39.3 g, yield 65.9%) was obtained using the same manner as in Step 1 of Preparation Example 1.
Mass : [(M+H)⁺] : 417

### <Step 2> Synthesis of Core 3

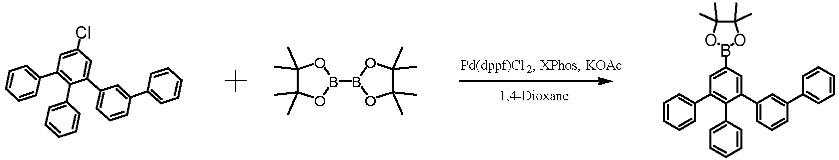

39.3 g(94.3 mmol) of 4'-chloro-6'-phenyl-1,1':2',1'':3'',1'''-quaterphenyl, 31.1 g(122.5 mmol) of 4,4,4',4',5,5,5',5'-octamethyl-2,2'-bi(1,3,2-dioxaborolane), 2.3 g(2.8 mmol) of Pd(dppf)Cl₂, 27.8 g (282.8 mmol) of KOAc, 2.7 g (5.7 mmol) of Xphos were added to 400 ml of 1,4-Dioxane, and Core3 (36.8 g, yield 76.7%) was obtained using the same manner as in Core 1 of Preparation Example 1.
Mass : [(M+H)⁺] : 508

### [Preparation Example 4]

### <Step 1> Synthesis of Core 4

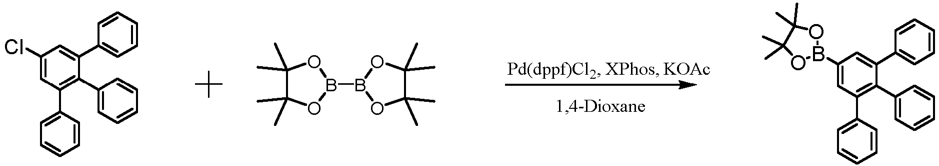

50.0 g(146.7 mmol) of 5'-chloro-3'-phenyl-1,1':2',1"-terphenyl, 48.4 g(190.7 mmol) of 4,4,4',4',5,5,5',5'-octamethyl-2,2'-bi(1,3,2-dioxaborolane), 3.6 g(4.4 mmol) of Pd(dppf)Cl₂, 43.2 g (440.1 mmol) of KOAc, and 4.2 g (8.8 mmol) of Xphos were added to 500 ml of 1,4-Dioxane, and Core 4 (51.12 g, yield 80.6%) was obtained using the same manner as in Core 1 of Preparation Example 1.
Mass : [(M+H)⁺] : 433

### [Preparation Example 5]

### <Step 1> Synthesis of Core 5

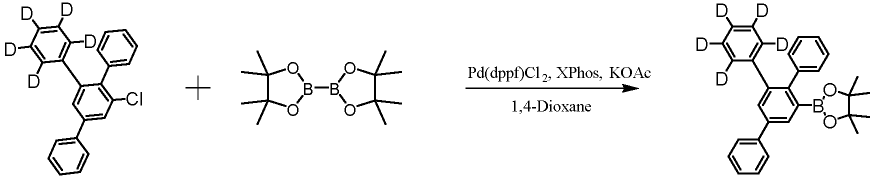

50.0 g(144.6 mmol) of 3'-chloro-5'-phenyl-1,1':2',1"-terphenyl-2,3,4,5,6-d5, 47.7 g(187.9 mmol) of 4,4,4',4',5,5,5',5'-octamethyl-2,2'-bi(1,3,2-dioxaborolane), 3.5 g(4.3 mmol) of Pd(dppf)Cl₂, 42.6 g (433.7 mmol) of KOAc, and 4.1 g (8.7 mmol)Xphos were added to 500 ml of 1,4-Dioxane 500 ml, Core 5 (31.7 g, yield 50.2%) was obtained using the same manner as in Core 1 of Preparation Example 1.
Mass : [(M+H)⁺] : 438

### [Preparation Example 6]

### <Step 1> Synthesis of 9-(3-chloro-5-(naphthalen-2-yl)phenyl)phenanthrene

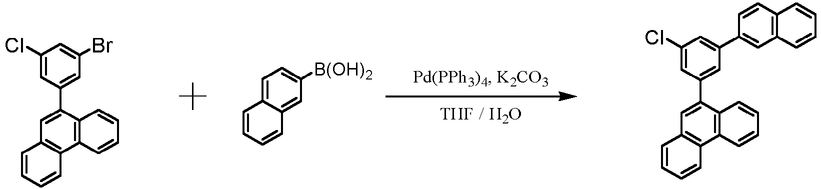

55 g(149.6 mmol) of 9-(3-bromo-5-chlorophenyl)phenanthrene, 28.3 g(164.6 mmol) of naphthalen-2-ylboronic acid, 5.2 g(4.5 mmol) of Pd(PPh₃)₄, and 62.0 g(448.8 mmol) of K₂CO₃ were added to 600 ml of THF, 150 ml of H₂O, and the target compound, 9-(3-chloro-5-(naphthalen-2-yl)phenyl)phenanthrene (42.3 g, yield 68.2%) was obtained by using the same manner as in Step 1 of Preparation Example 1.
Mass : [(M+H)⁺] : 415

### <Step 1> Synthesis of Core6

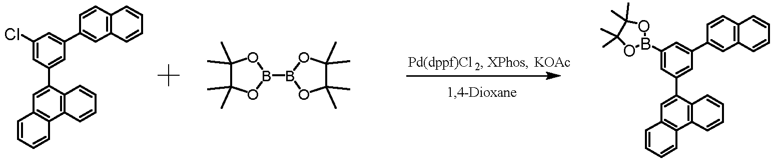

42.3 g(101.9 mmol) of 9-(3-chloro-5-(naphthalen-2-yl)phenyl)phenanthrene, 33.7 g(132.5 mmol) of 4,4,4',4',5,5,5',5'-octamethyl-2,2'-bi(1,3,2-dioxaborolane), 2.5 g(4.7 mmol) of Pd(dppf)Cl₂, 30.0 g (305.8 mmol) of KOAc, 2.9 g (9.5 mmol) of Xphos were added to 450 ml of 1,4-Dioxane 450 ml, and Core6 (31.6 g, yield 61.2%) was obtained by using the same manner as in Core 1 of Preparation Example 1
Mass : [(M+H)⁺] : 506

### [Preparation Example 7]

### <Step 1> Synthesis of 5'-chloro-1,1':3',1":3",1‴-quaterphenyl

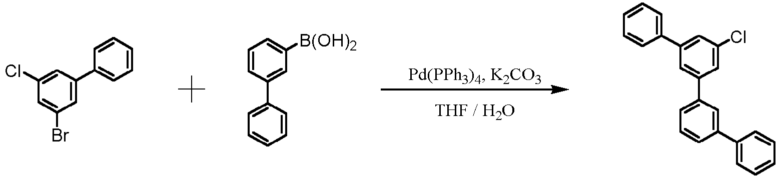

70 g(261.6 mmol) of 3-bromo-5-chloro-1,1'-biphenyl, 54.4 g(274.7 mmol) of [1,1'-biphenyl]-3-ylboronic acid, 9.1 g(7.8 mmol) of Pd(PPh₃)₄, 108.5 g(784.9 mmol) of K₂CO₃ were added to 870 ml of THF, 220 ml of H₂O, the target compound, 5'-chloro-1,1':3',1":3",1‴-quaterphenyl (74.1 g, yield 83.1%) was obtained by using the same manner as in Step 1 of Preparation Example 1.
Mass : [(M+H)⁺] : 341

### <Step 1> Synthesis of Core7

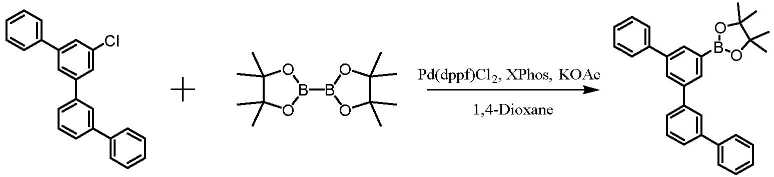

74.1 g(221.7 mmol) of 5'-chloro-1,1':3',1":3",1‴-quaterphenyl, 71.8 g(282.6 mmol) of 4,4,4',4',5,5,5',5'-octamethyl-2,2'-bi(1,3,2-dioxaborolane), 5.3 g(6.5 mmol) of Pd(dppf)Cl₂, 64.0 g (652.2 mmol) of KOAc, 6.2 g (13.0 mmol) of Xphos were added to 750 ml of 1,4-Dioxane 750 ml, and Core7 (77.2 g, yield 77.1%) was obtained by using the same manner as in Core 1 of Preparation Example 1.
Mass : [(M+H)⁺] : 433

### [Preparation Example 8]

### <Step 1> Synthesis of 5'-chloro-1,1':3',1":4",1‴-quaterphenyl

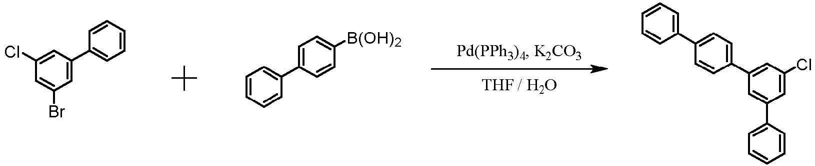

70 g(261.6 mmol) of 3-bromo-5-chloro-1,1'-biphenyl, 57.0 g(287.8 mmol) of [1,1'-biphenyl]-4-ylboronic acid, 9.1 g(7.8 mmol) of Pd(PPh₃)₄, 108.5 g(784.9 mmol) of K₂CO₃ were added to 700 ml of THF 700 ml, and 185 ml of H₂O, and the target compound, 5'-chloro-1,1':3',1":4",1‴-quaterphenyl (82.1 g, yield 92.1%) was obtained by using the same manner as in Step 1 of Preparation Example 1.
Mass : [(M+H)⁺] : 341

### <Step 1> Synthesis of Core8

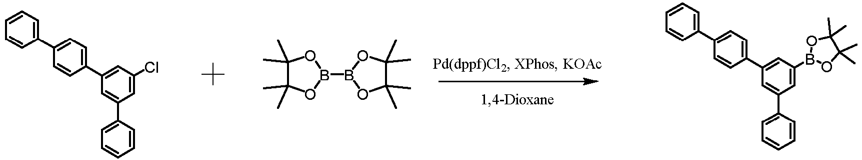

82.1 g(220.9 mmol) of 5'-chloro-1,1':3',1'':4'',1‴-quaterphenyl, 79.5 g(313.1 mmol) of 4,4,4',4',5,5,5',5'-octamethyl-2,2'-bi(1,3,2-dioxaborolane), 5.9 g(7.2 mmol) of Pd(dppf)Cl₂, 70.9 g (722.6 mmol) of KOAc, 6.9 g (14.4 mmol) of Xphos were added to 750 ml of 1,4-Dioxane, and Core8 (91.2 g, yield 87.6%) was obtained by using the same manner as in Core 1 of Preparation Example 1.

### [Preparation Example 9]

### <Step 1> Synthesis of 2-(5'-chloro-[1,1':3',1''-terphenyl]-4-yl)naphthalene

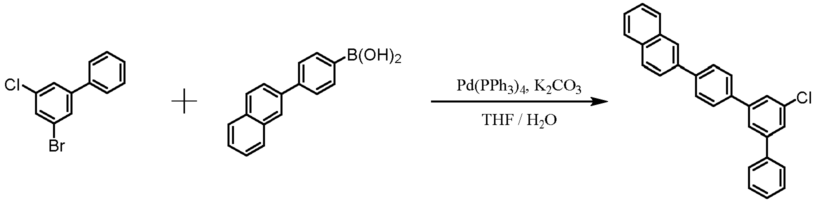

70 g(261.6 mmol) of 3-bromo-5-chloro-1,1'-biphenyl, 71.4 g(287.8 mmol) of (4-(naphthalen-2-yl)phenyl)boronic acid, 9.1 g(7.8 mmol) of Pd(PPh₃)₄, and 108.5 g(784.9 mmol) of K₂CO₃ were added to 870 ml of THF, 220 ml of H₂O 220 ml, and the target compound, 2-(5'-chloro-[1,1':3',1"-terphenyl]-4-yl)naphthalene (88.8 g, yield 86.8%) was obtained by using the same manner as in Step 1 of Preparation Example 1.
Mass : [(M+H)⁺] : 391

### <Step 1> Synthesis of Core9

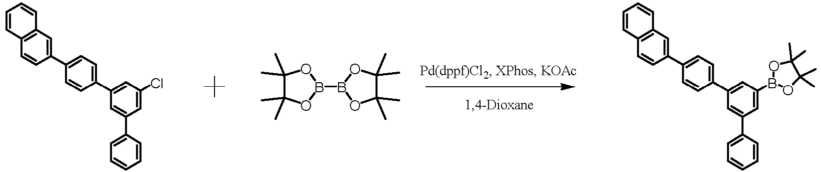

88.8 g(227.2 mmol) of 2-(5'-chloro-[1,1':3',1''-terphenyl]-4-yl)naphthalene, 75.0 g(381.4 mmol) of 4,4,4',4',5,5,5',5'-octamethyl-2,2'-bi(1,3,2-dioxaborolane), 5.6 g(6.8 mmol) of Pd(dppf)Cl₂, 66.9 g (681.5 mmol) of KOAc, and 6.5 g (13.6 mmol) of Xphos were added to 800 ml of 1,4-Dioxane, and Core9 (94.5 g, yield 82.1%) was obtained by using the same manner as in Core 1 of Preparation Example 1.
Mass : [(M+H)⁺] : 433

### [Synthesis Example 1] Synthesis of Compound 1

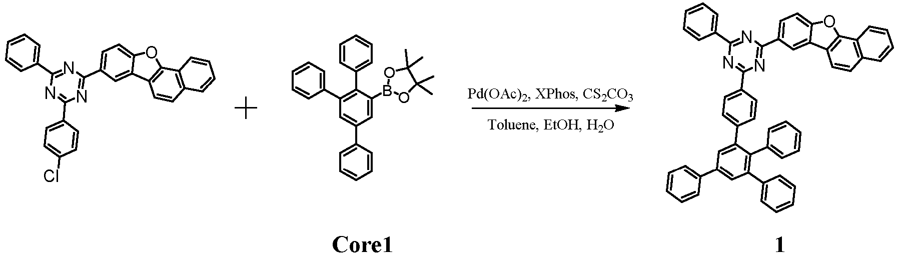

15.0 g(1eq, 31.0 mmol) of 2-(4-chlorophenyl)-4-(naphtho[1,2-b]benzofuran-8-yl)-6-phenyl-1,3,5-triazine, 14.1 g(1.05eq, 32.5 mmol) of Core 1 in Preparation Example 1, 0.2 g(0.03eq, 0.9 mmol) of Pd(OAc)₂, 30.3 g(3.0eq, 93.0 mmol) of Cs₂CO₃, and 0.9 g(0.06eq, 1.9 mmol) of Xphos were added to 200 ml of Toluene, 50 ml of EtOH, and 50 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 1 (18.6 g, yield 78.2%).
Mass : [(M+H)⁺] : 753

### [Synthesis Example 2] Synthesis of Compound 3

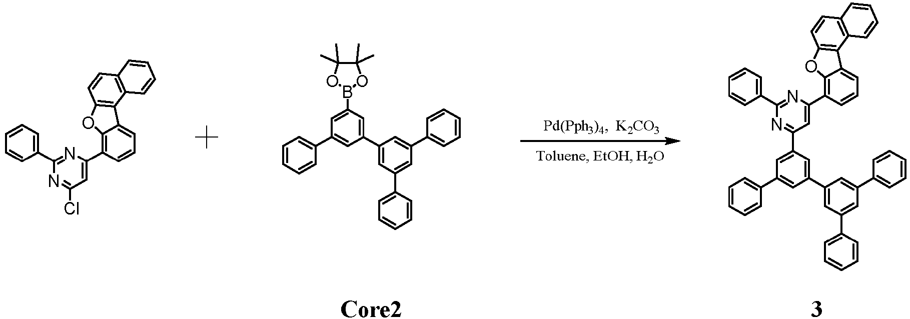

17.0 g(1eq, 41.8 mmol) of 4-chloro-6-(naphtho[2,1-b]benzofuran-8-yl)-2-phenylpyrimidine, 22.3 g(1.05eq, 43.9 mmol) of Core2 in Preparation Example 2, 2.1 g(0.05eq, 1.3 mmol) of Pd(Pph₃)₄, and 17.3 g(3.0eq, 125.4 mmol) of K₂CO₃ were added to 200 ml of Toluene, 50 ml of EtOH, and 50 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 3 (22.4 g, yield 71.1 %) .
Mass : [(M+H)⁺] : 753

### [Synthesis Example 3] Synthesis of Compound 6

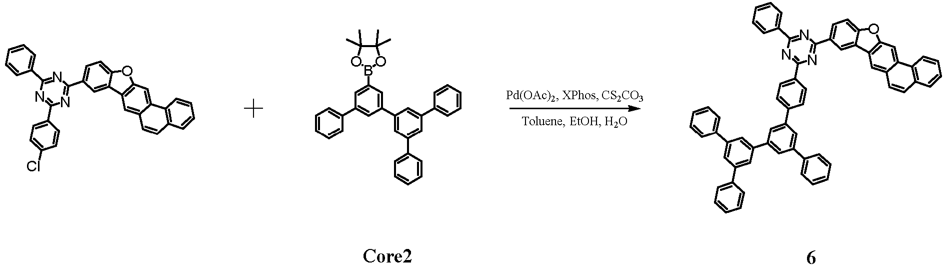

10.0 g(1eq, 18.7 mmol) of 2-(4-chlorophenyl)-4-(phenanthro[3,2-b]benzofuran-9-yl)-6-phenyl-1,3,5-triazine, 10.0 g(1.05eq, 19.7 mmol) of Core2 in Preparation Example 2, 0.1 g(0.03eq, 0.56 mmol) of Pd(OAc)₂, 18.3 g(3.0eq, 56.2 mmol) of Cs₂CO₃, 0.5 g(0.06eq, 1.1 mmol) of Xphos were added to 200 ml of Toluene, 50 ml of EtOH, 50 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 6 (13.5 g, yield 81.9 %).
Mass : [(M+H)⁺] : 881

### [Synthesis Example 4] Synthesis of Compound 10

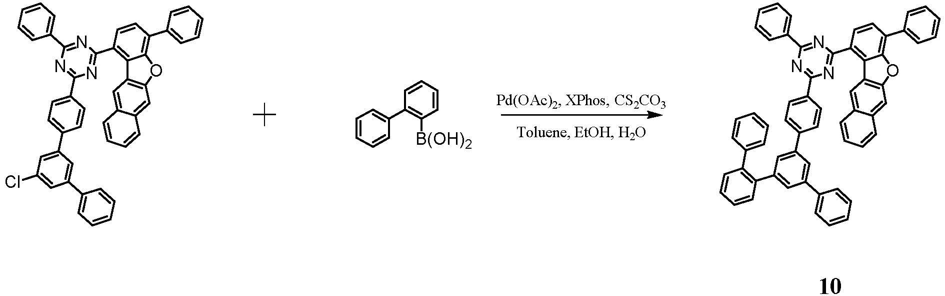

10.0 g(1eq, 14.0 mmol) of 2-(5'-chloro-[1,1':3',1"-terphenyl]-4-yl)-4-phenyl-6-(4-phenylnaphtho[2,3-b]benzofuran-1-yl)-1,3,5-triazine, 2.9 g(1.05eq, 14.74 mmol) of [1,1'-biphenyl]-2-ylboronic acid, 0.1 g(0.03eq, 0.42 mmol) of Pd(OAc)₂, 13.7 g(3.0eq, 42.1 mmol) of Cs₂CO₃, and 0.4 g(0.06eq, 0.8 mmol) of Xphos were added to 200 ml of Toluene 200ml, 50 ml of EtOH 50ml, and 50 ml of H₂O 50ml, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 10 (4.4 g, yield 38.2 %).
Mass : [(M+H)⁺] : 831

### [Synthesis Example 5] Synthesis of Compound 11

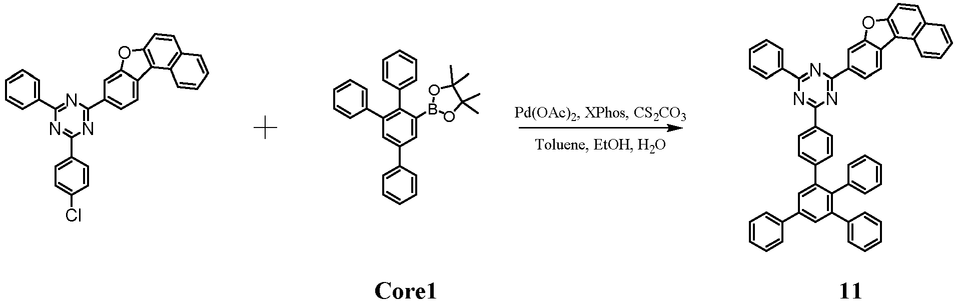

Except that 15.0 g (35.1 mmol) of 2-(4-chlorophenyl)-4-(naphtho2,1-bbenzofuran-9-yl)-6-phenyl-1,3,5-triazine was used instead of 2-(4-chlorophenyl)-4-(naphthol,2-bbenzofuran-8-yl)-6-phenyl-1,3,5-triazine, the remaining reagents were used in the same equivalent ratio as in Synthesis Example 1 and the same procedure was carried out to obtain target compound, Compound 11 (22.9 g, yield 86.3%).
Mass : [(M+H)⁺] : 754

### [Synthesis Example 6] Synthesis of Compound 23

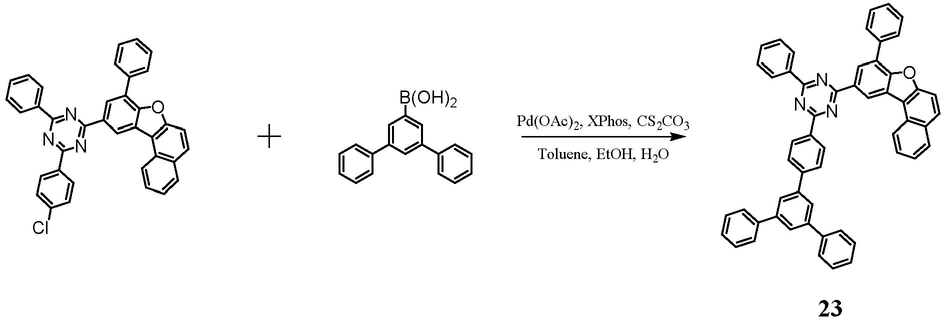

12.0 g(1eq, 21.4 mmol) of 2-(4-chlorophenyl)-4-phenyl-6-(8-phenylnaphtho[2,1-b]benzofuran-10-yl)-1,3,5-triazine, 6.2 g(1.05eq, 22.5 mmol) of [1,1':3',1"-terphenyl]-5'-ylboronic acid, 0.1 g(0.03eq, 0.64 mmol) of Pd(OAc)₂, 20.9 g(3.0eq, 64.3 mmol) of Cs₂CO₃, and 0.6 g(0.06eq, 1.3 mmol) of Xphos were added to 200 ml of Toluene 200ml, 50 ml of EtOH, 50 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 23 (13.9 g, yield 85.8 %).
Mass : [(M+H)⁺] : 755

### [Synthesis Example 7] Synthesis of Compound 27

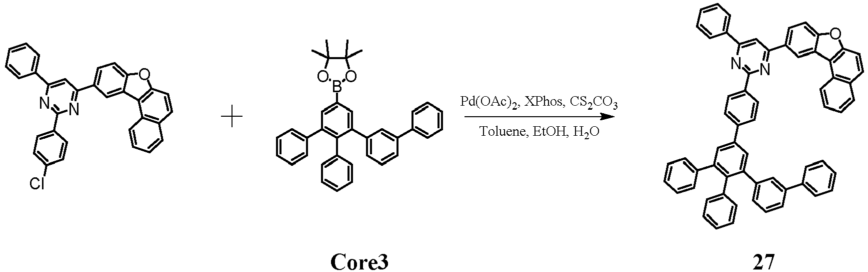

13.0 g(1eq, 26.9 mmol) of 2-(4-chlorophenyl)-4-(naphtho[2,1-b]benzofuran-10-yl)-6-phenylpyrimidine, 14.4 g(1.05eq, 28.3 mmol) of Core3 in Preparation Example 3, 0.2 g(0.03eq, 0.81 mmol) of Pd(OAc)₂, 26.3 g(3.0eq, 80.8 mmol) of Cs₂CO₃, 0.8 g(0.06eq, 1.6 mmol) of Xphos were added to 200 ml of Toluene, 50 ml of EtOH, 50 ml of H₂O, and the mixture was heated under reflux for 6 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 27 (16.8 g, yield 75.2 %).
Mass : [(M+H)⁺] : 829

### [Synthesis Example 8] Synthesis of Compound 28

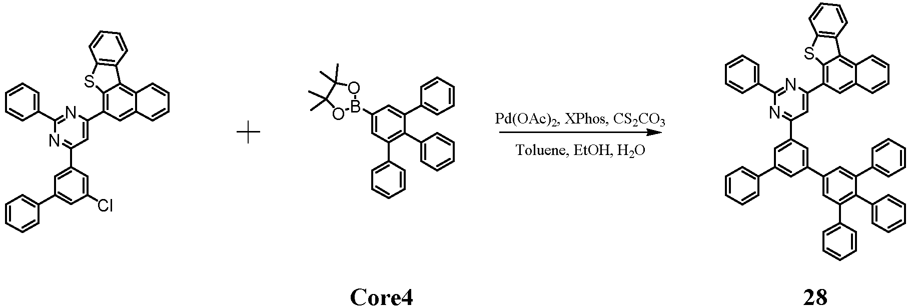

20.0 g(1eq, 34.8 mmol) of 4-(benzo[b]naphtho[1,2-d]thiophen-6-yl)-6-(5-chloro-[1,1'-biphenyl]-3-yl)-2-phenylpyrimidine, 15.8 g(1.05eq, 36.5 mmol) of Core4 in Preparation Example 4, 0.2 g(0.03eq, 1.0 mmol) of Pd(OAc)₂, 34.0 g(3.0eq, 104.3 mmol) of Cs₂CO₃, 1.0 g(0.06eq, 2.1 mmol) of Xphos were added to 300 ml of Toluene, 75 ml of EtOH, 75 ml of H₂O, and the mixture was heated under reflux for 6 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 28 (17.9 g, yield 61.1 %).
Mass : [(M+H)⁺] : 845

### [Synthesis Example 9] Synthesis of Compound 37

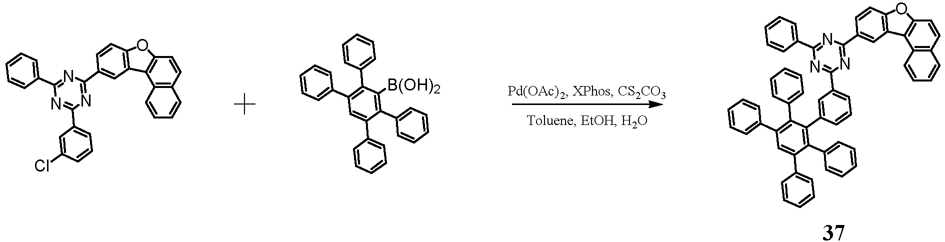

10.0 g(1eq, 20.7 mmol) of 2-(3-chlorophenyl)-4-(naphtho[2,1-b]benzofuran-10-yl)-6-phenyl-1,3,5-triazine, 9.2 g(1.05eq, 21.7 mmol) of (4',5'-diphenyl-[1,1':2',1''-terphenyl]-3'-yl)boronic acid, 0.1 g(0.03eq, 0.62 mmol) of Pd(OAc)₂, 20.2 g(3.0eq, 61.9 mmol) of Cs₂CO₃, 0.6g (0.06eq, 1.2 mmol) of Xphos were added to 200 ml of Toluene, 50 ml of EtOH, 50 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 37 (6.7 g, yield 38.9 %).
Mass : [(M+H)⁺] : 831

### [Synthesis Example 10] Synthesis of Compound 38

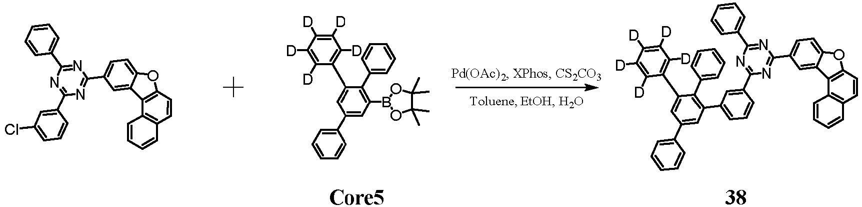

25.0 g(1eq, 51.7 mmol) of 2-(3-chlorophenyl)-4-(naphtho[2,1-b]benzofuran-10-yl)-6-phenyl-1,3,5-triazine, 23.7 g(1.05eq, 54.7 mmol) of Core5 in Preparation Example 5, 0.4 g(0.03eq, 1.6 mmol) of Pd(OAc)₂, 50.5 g(3.0eq, 155.0 mmol) of Cs₂CO₃, 1.5 g(0.06eq, 3.1 mmol) of Xphos were added to 300 ml of Toluene, 75 ml of EtOH, 75 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 38 (31.3 g, yield 79.9 %)
Mass : [(M+H)⁺] : 759

### [Synthesis Example 11] Synthesis of Compound 39

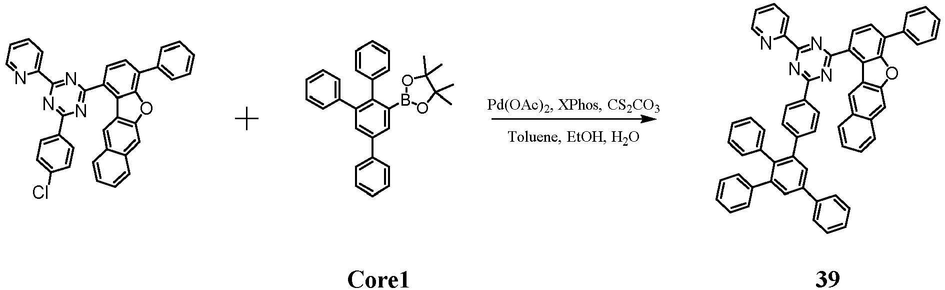

Except that 10.0 g(17.8 mmol) of 2-(4-chlorophenyl)-4-(4-phenylnaphtho[2,3-b]benzofuran-1-yl)-6-(pyridin-2-yl)-1,3,5-triazine was used instead of 2-(4-chlorophenyl)-4-(naphtho1,2-bbenzofuran-8-yl)-6-phenyl-1,3,5-triazine, the remaining reagents were used in the same equivalent ratio as in Synthesis Example 1 and the same procedure was carried out to obtain target compound, Compound 39 (13.1 g, yield 88.3%).
Mass : [(M+H)⁺] : 832

### [Synthesis Example 12] Synthesis of Compound 41

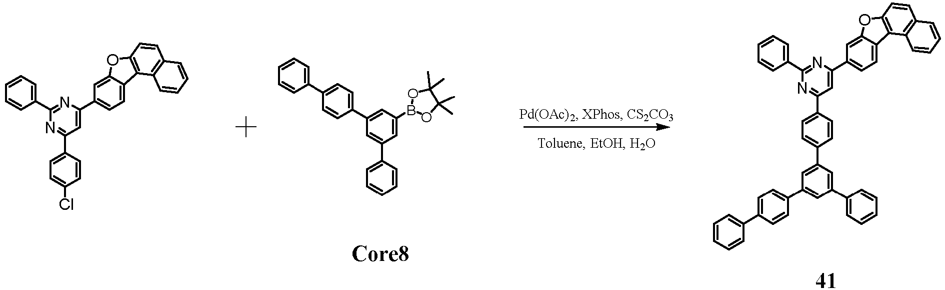

15.0 g(1eq, 31.1 mmol) of 4-(4-chlorophenyl)-6-(naphtho[2,1-b]benzofuran-9-yl)-2-phenylpyrimidine, 14.1 g(1.05eq, 32.6 mmol) of Core8 in Preparation Example 8, 0.2 g(0.03eq, 0.9 mmol) of Pd(OAc)₂, 30.4 g(3.0eq, 93.2 mmol) of Cs₂CO₃, 0.9 g(0.06eq, 1.9 mmol) of Xphos were added to 300 ml of Toluene, 75 ml of EtOH, 75 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 41 (19.7 g, yield 84.2 %).
Mass : [(M+H)⁺] : 754

### [Synthesis Example 13] Synthesis of Compound 45

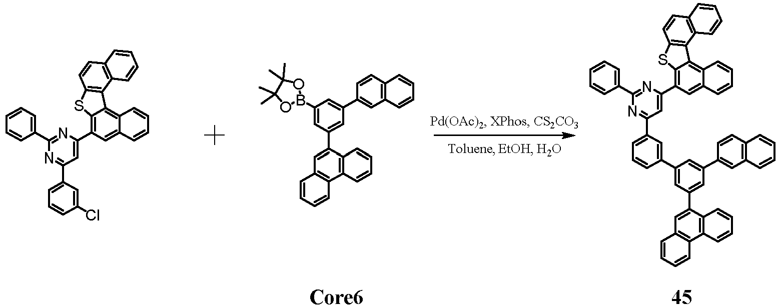

20.0 g(1eq, 36.4 mmol) of 4-(3-chlorophenyl)-6-(dinaphtho[2,1-b:1',2'-d]thiophen-6-yl)-2-phenylpyrimidine, 19.4 g(1.05eq, 38.3 mmol) of Core6 in Preparation Example 6, 0.3 g(0.03eq, 1.1 mmol) of Pd(OAc)₂, 35.6 g(3.0eq, 109.3 mmol) of Cs₂CO₃, 1.0 g(0.06eq, 2.2 mmol) of Xphos were added to 300 ml of Toluene, 75 ml of EtOH, 75 ml of H₂O, and the mixture was heated under reflux for 6 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 45 (19.4 g, yield 59.7 %).
Mass : [(M+H)⁺] : 893

### [Synthesis Example 14] Synthesis of Compound 48

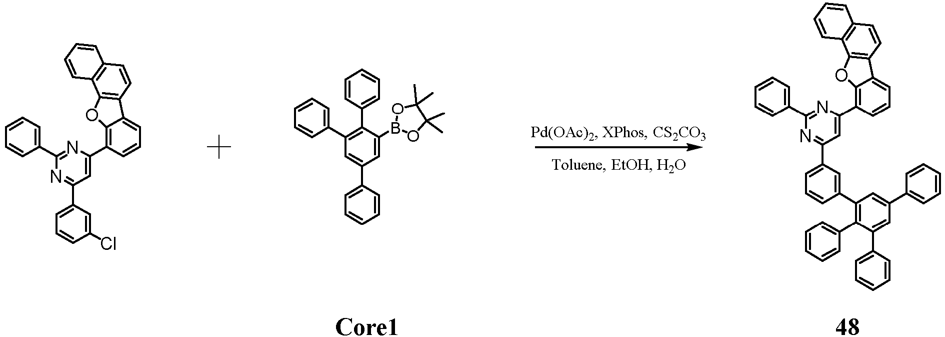

15.0 g(1eq, 31.1 mmol) of 4-(3-chlorophenyl)-6-(naphtho[1,2-b]benzofuran-10-yl)-2-phenylpyrimidine, 14.1 g(1.05eq, 32.6 mmol) of Core1 in Preparation Example 1, 0.2 g(0.03eq, 0.9 mmol) of Pd(OAc)₂, 30.4 g(3.0eq, 93.2 mmol) of Cs₂CO₃, 0.9 g(0.06eq, 1.9 mmol) of Xphos were added to 300 ml of Toluene, 75 ml of EtOH, 75 ml of H₂O, and the mixture was heated under reflux for 6 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 48 (19.2 g, yield 82.1 %).
Mass : [(M+H)⁺] : 754

### [Synthesis Example 15] Synthesis of Compound 50

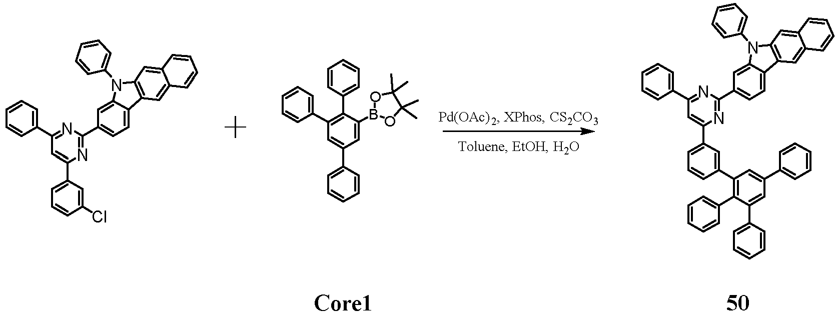

Except that 15.0 g (26.9 mmol) of 3-(4-(3-chlorophenyl)-6-phenylpyrimidin-2-yl)-5-phenyl-5H-benzo[b]carbazole was used instead of 2-(4-chlorophenyl)-4-(naphtho1,2-bbenzofuran-8-yl)-6-phenyl-1,3,5-triazine, the remaining reagents were used in the same equivalent ratio as in Synthesis Example 1 and the same procedure was carried out to obtain target compound, Compound 50 (17.1 g, yield 76.6%).
Mass : [(M+H)⁺] : 829

### [Synthesis Example 16] Synthesis of Compound 51

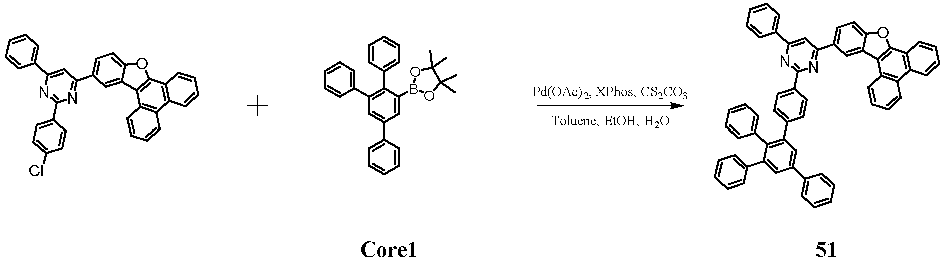

Except that 15.0 g (28.1 mmol) of 2-(4-chlorophenyl)-4-(phenanthro[9,10-b]benzofuran-12-yl)-6-phenylpyrimidine was used instead of 2-(4-chlorophenyl)-4-(naphtho1,2-bbenzofuran-8-yl)-6-phenyl-1,3,5-triazine, the remaining reagents were used in the same equivalent ratio as in Synthesis Example 1 and the same procedure was carried out to obtain target compound, Compound 51 (20.9 g, yield 92.3%).
Mass : [(M+H)⁺] : 804

### [Synthesis Example 17] Synthesis of Compound 54

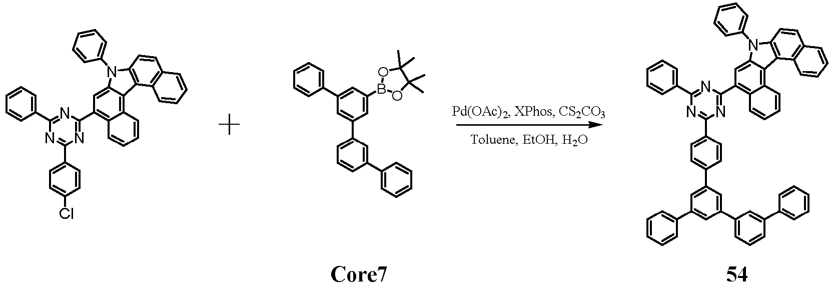

16.0 g(1eq, 26.3 mmol) of 5-(4-(4-chlorophenyl)-6-phenyl-1,3,5-triazin-2-yl)-7-phenyl-7H-dibenzo[c,g]carbazole, 11.9 g(1.05eq, 27.6 mmol) of Core7 in Preparation Example 7, 0.2 g(0.03eq, 0.8 mmol) of Pd(OAc)₂, 25.7 g(3.0eq, 78.8 mmol) of Cs₂CO₃, 0.7 g(0.06eq, 1.6 mmol) of Xphos were added to 200 ml of Toluene, 50 ml of EtOH, 50 ml of H₂O, and the mixture was heated under reflux for 8 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 54 (15.3 g, yield 66.3 %).
Mass : [(M+H)⁺] : 879

### [Synthesis Example 18] Synthesis of Compound 57

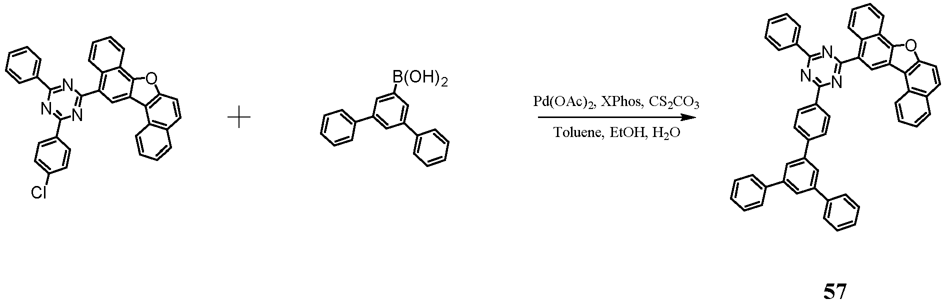

13.0 g(1eq, 24.3 mmol) of 2-(4-chlorophenyl)-4-(dinaphtho[1,2-b:1',2'-d]furan-12-yl)-6-phenyl-1,3,5-triazine, 7.0 g(1.05eq, 25.6 mmol) of [1,1':3',1"-terphenyl]-5'-ylboronic acid, 0.2 g(0.03eq, 0.7 mmol) of Pd(OAc)₂, 23.8 g(3.0eq, 73.0 mmol) of Cs₂CO₃, 0.7 g(0.06eq, 1.5 mmol) of Xphos were added to 400 ml of Toluene, 100 ml of EtOH, 100 ml of H₂O, and the mixture was heated under reflux for 8 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 57 (15.7 g, yield 88.8 %).
Mass : [(M+H)⁺] : 729

### [Synthesis Example 19] Synthesis of Compound 59

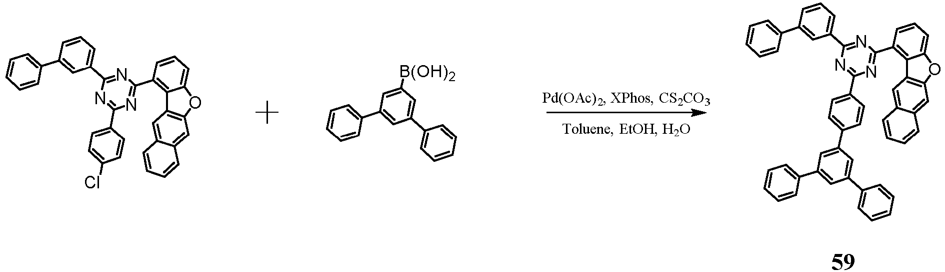

10.0 g(1eq, 17.9 mmol) of 2-([1,1'-biphenyl]-3-yl)-4-(4-chlorophenyl)-6-(naphtho[2,3-b]benzofuran-1-yl)-1,3,5-triazine, 5.1 g(1.05eq, 18.7 mmol) of [1,1':3',1"-terphenyl]-5'-ylboronic acid, 0.1 g(0.03eq, 0.5 mmol) of Pd(OAc)₂, 17.5 g(3.0eq, 53.6 mmol) of Cs₂CO₃, 0.5 g(0.06eq, 1.1 mmol) of Xphos were added to 300 ml of Toluene, 75 ml of EtOH, 75 ml of H₂O, and the mixture was heated under reflux for 8 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 59 (8.8 g, yield 65.7 %).
Mass : [(M+H)⁺] : 755

### [Synthesis Example 20] Synthesis of Compound 67

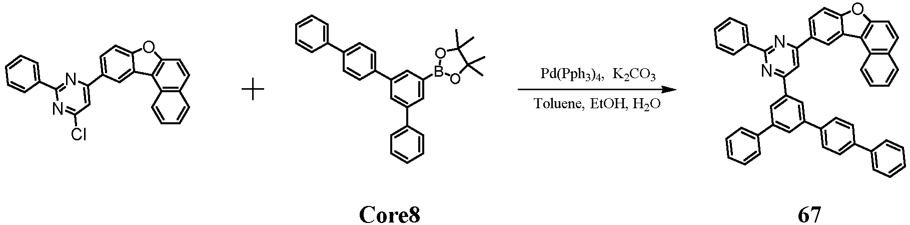

20.0 g(1eq, 49.2 mmol) of 4-chloro-6-(naphtho[2,1-b]benzofuran-10-yl)-2-phenylpyrimidine, 22.3 g(1.05eq, 51.6 mmol) of Core8 in Preparation Example 8, 2.1 g(0.05eq, 2.5 mmol) of Pd(Pph₃)₄, 20.4 g(3.0eq, 147.5 mmol) of K₂CO₃ were added to 300 ml of Toluene 300ml, 75 ml of EtOH, 75 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 67 (28.2 g, yield 84.8 %).
Mass : [(M+H)⁺] : 677

### [Synthesis Example 21] Synthesis of Compound 72

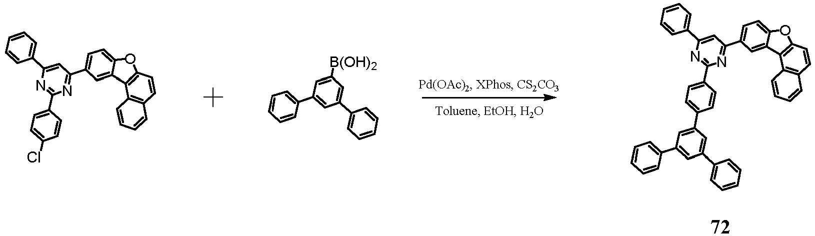

20.0 g(1eq, 41.4 mmol) of 2-(4-chlorophenyl)-4-(naphtho[2,1-b]benzofuran-10-yl)-6-phenylpyrimidine, 11.9 g(1.05eq, 43.5 mmol) of [1,1':3',1"-terphenyl]-5'-ylboronic acid, 0.3 g(0.03eq, 1.2 mmol) of Pd(OAc)₂, 40.5 g(3.0eq, 124.2 mmol) of Cs₂CO₃, 1.2 g(0.06eq, 2.5 mmol) of Xphos were added to 200 ml of Toluene, 50 ml of EtOH, 50 ml of H₂O, and the mixture was heated under reflux for 8 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 72 (22.9 g, yield 81.9 %).
Mass : [(M+H)⁺] : 677

### [Synthesis Example 22] Synthesis of Compound 76

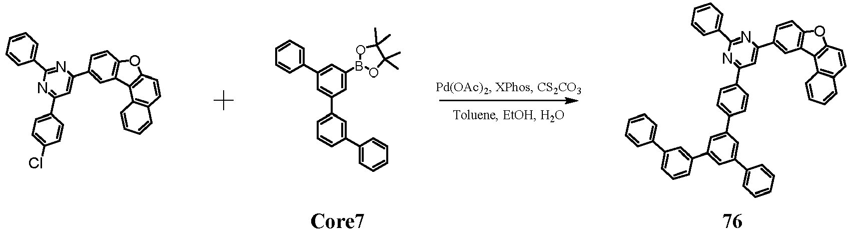

Except that 15.0 g (35.1 mmol) of 4-(4-chlorophenyl)-6-(naphtho[2,1-b]benzofuran-10-yl)-2-phenylpyrimidine was used instead of 5-(4-(4-chlorophenyl)-6-phenyl-1,3,5-triazin-2-yl)-7-phenyl-7H-dibenzo[c,g]carbazole, the remaining reagents were used in the same equivalent ratio as in Synthesis Example 9 and the same procedure was carried out to obtain target compound, Compound 76 (23.6 g, yield 75.8%).
Mass : [(M+H)⁺] : 753

### [Synthesis Example 23] Synthesis of Compound 80

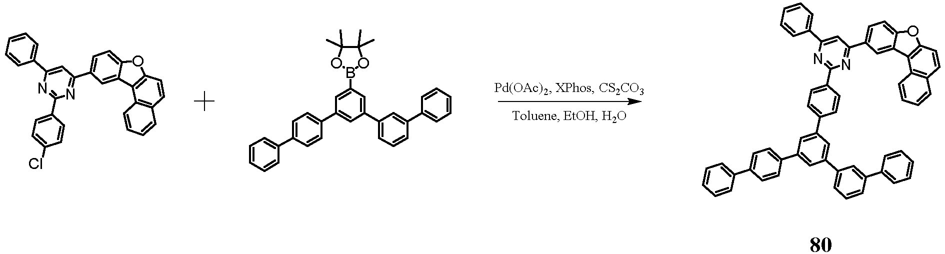

10.0 g(1eq, 20.7 mmol) of 2-(4-chlorophenyl)-4-(naphtho[2,1-b]benzofuran-10-yl)-6-phenylpyrimidine, 11.1 g(1.05eq, 21.7 mmol) of 2-([1,1':3',1":3",1‴:4‴,1ʺʺ-quinquephenyl]-5"-yl)-4,4,5,5-tetramethyl-1,3,2-dioxaborolane, 0.1 g(0.03eq, 0.62mmol) of Pd(OAc)₂, 20.2 g(3.0eq, 62.1 mmol) of Cs₂CO₃, 0.6 g(0.06eq, 1.2 mmol) of Xphos were added to 200 ml of Toluene, 50 ml of EtOH, 50 ml of H₂O, and the mixture was heated under reflux for 7 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 80 (12.7 g, yield 74.4 %).
Mass : [(M+H)⁺] : 830

### [Synthesis Example 24] Synthesis of Compound 81

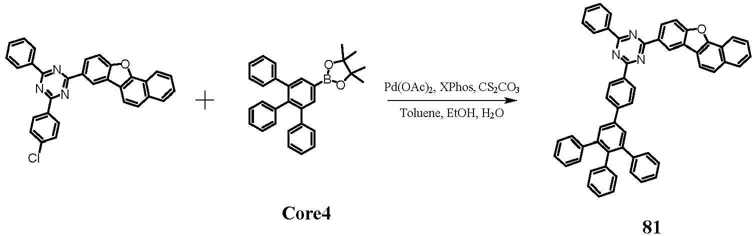

Except that 15.0 g (31.0 mmol) of 2-(4-chlorophenyl)-4-(naphtho[1,2-b]benzofuran-8-yl)-6-phenyl-1,3,5-triazine was used instead of 4-(benzo[b]naphtho[1,2-d]thiophen-6-yl)-6-(5-chloro-[1,1'-biphenyl]-3-yl)-2-phenylpyrimidine, the remaining reagents were used in the same equivalent ratio as in Synthesis Example 6 and the same procedure was carried out to obtain target compound, Compound 81 (19.2 g, yield 82.2%).
Mass : [(M+H)⁺] : 755

### [Synthesis Example 25] Synthesis of Compound 82

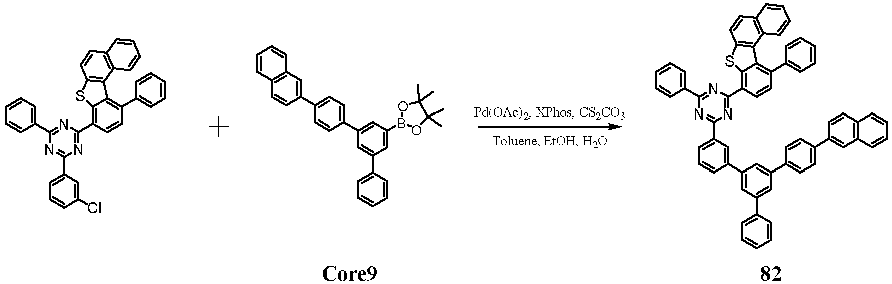

17.0 g(1eq, 29.5 mmol) of 2-(3-chlorophenyl)-4-phenyl-6-(11-phenylbenzo[b]naphtho[1,2-d]thiophen-8-yl)-1,3,5-triazine, 14.9 g(1.05eq, 31.0 mmol) of Core9 in Preparation Example 9, 0.9 g(0.03eq, 0.89mmol) of Pd(OAc)₂, 28.8 g(3.0eq, 74.1 mmol) of Cs₂CO₃, 0.8 g(0.06eq, 1.8 mmol) of Xphos were added to 200 ml of Toluene, 50 ml of EtOH, 50 ml of H₂O, and the mixture was heated under reflux for 7 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 82 (16.3 g, yield 61.8 %).
Mass : [(M+H)⁺] : 896

### [Synthesis Example 26] Synthesis of Compound 83

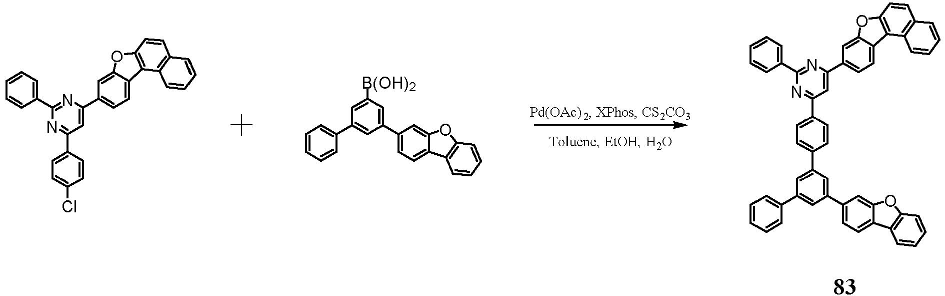

10.0 g(1eq, 20.7 mmol) of 4-(4-chlorophenyl)-6-(naphtho[2,1-b]benzofuran-9-yl)-2-phenylpyrimidine, 9.9 g(1.05eq, 21.7 mmol) of (5-(dibenzo[b,d]furan-3-yl)-[1,1'-biphenyl]-3-yl)boronic acid, 0.1 g(0.03eq, 0.62mmol) of Pd(OAc)₂, 20.2 g(3.0eq, 62.1 mmol) of Cs₂CO₃, 0.6 g(0.06eq, 1.2 mmol) of Xphos were added to 200 ml of Toluene, 50 ml of EtOH, 50 ml of H₂O, and the mixture was heated under reflux for 7 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 83 (9.9 g, yield 62.1 %).
Mass : [(M+H)⁺] : 768

### [Synthesis Example 27] Synthesis of Compound 84

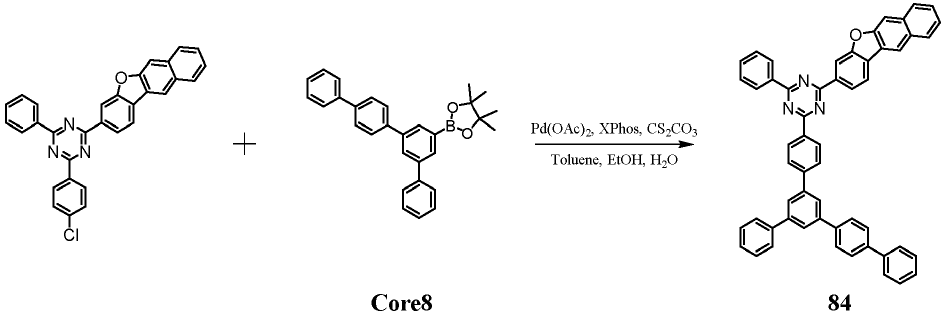

12.0 g(1eq, 24.8 mmol) of 2-(4-chlorophenyl)-4-(naphtho[2,3-b]benzofuran-3-yl)-6-phenyl-1,3,5-triazine, 11.3 g(1.05eq, 26.0 mmol) of Core8 in Preparation Example 8, 0.2 g(0.03eq, 0.74mmol) of Pd(OAc)₂, 24.2 g(3.0eq, 74.4 mmol) of Cs₂CO₃, 0.7 g(0.06eq, 1.5 mmol) of Xphos were added to 400 ml of Toluene, 100 ml of EtOH, 100 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 84 (17.1 g, yield 91.2 %).
Mass : [(M+H)⁺] : 755

### [Synthesis Example 28] Synthesis of Compound 89

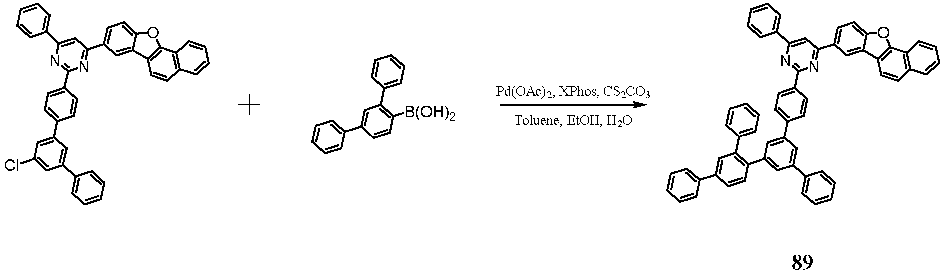

10.0 g(1eq, 15.7 mmol) of 2-(5'-chloro-[1,1':3',1''-terphenyl]-4-yl)-4-(naphtho[1,2-b]benzofuran-8-yl)-6-phenylpyrimidine, 4.5 g(1.05eq, 16.5 mmol) of [1,1':3',1"-terphenyl]-4'-ylboronic acid, 0.1 g(0.03eq, 0.47mmol) of Pd(OAc)₂, 15.4 g(3.0eq, 47.2 mmol) of Cs₂CO₃, 0.4 g(0.06eq, 0.9 mmol) of Xphos were added to 200 ml of Toluene, 50 ml of EtOH, 50 ml of H₂O, and the mixture was heated under reflux for 6 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 89 (5.9 g, yield 44.9 %).
Mass : [(M+H)⁺] : 830

### [Synthesis Example 29] Synthesis of Compound 94

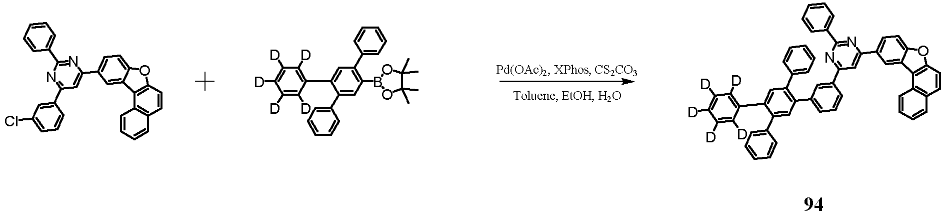

10.0 g(1eq, 20.7 mmol) of 4-(3-chlorophenyl)-6-(naphtho[2,1-b]benzofuran-10-yl)-2-phenylpyrimidine, 9.5 g(1.05eq, 21.7 mmol) of 4,4,5,5-tetramethyl-2-(5'-phenyl-[1,1':2',1"-terphenyl]-4'-yl-2,3,4,5,6-d5)-1,3,2-dioxaborolane, 0.1 g(0.03eq, 0.62mmol) of Pd(OAc)₂, 20.2 g(3.0eq, 62.1 mmol) of Cs₂CO₃, 0.6 g(0.06eq, 1.2 mmol) of Xphos were added to 200 ml of Toluene, 50 ml of EtOH, 50 ml of H₂O, and the mixture was heated under reflux for 6 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 94 (14.0 g, yield 89.3 %).
Mass : [(M+H)⁺] : 759

### [Synthesis Example 30] Synthesis of Compound 95

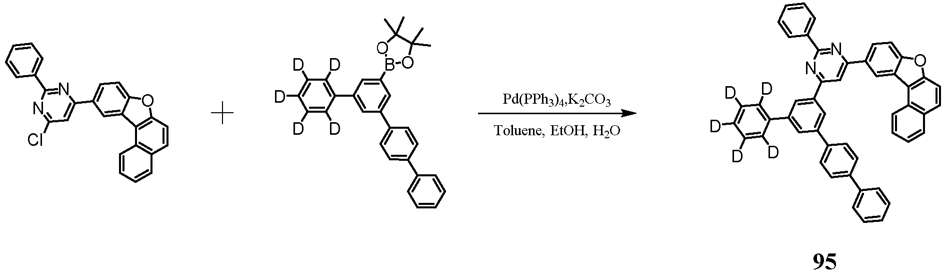

13.0 g(1eq, 31.9 mmol) of 4-chloro-6-(naphtho[2,1-b]benzofuran-10-yl)-2-phenylpyrimidine, 14.7 g(1.05eq, 33.6 mmol) of 2-([1,1':3',1":4",1‴-quaterphenyl]-5'-yl-2,3,4,5,6-d5)-4,4,5,5-tetramethyl-1,3,2-dioxaborolane, 1.8 g(0.05eq, 1.6 mmol) of Pd(Pph₃)₄, and 13.2 g(3.0eq, 95.8 mmol) of K₂CO₃ were added to 200 ml of Toluene, 50 ml of EtOH, 50 ml of H₂O, and the mixture was heated under reflux for 4 hours. After the reaction was completed, the mixture was extracted with dichloromethane, and MgSO₄ was added to remove moisture, followed by filtration, and then the solvent of the filtered organic layer was removed, and the product was purified by column chromatography using dichloromethane and hexane, and recrystallized with toluene, acetone to obtain the target compound, Compound 95 (18.8 g, yield 86.4 %).
Mass : [(M+H)⁺] : 683

### [Examples 1 to 10] Manufacturing of Blue organic EL elements

The compounds synthesized in the above synthesis examples were subjected to high-purity sublimation purification in a conventionally known method, and then blue organic EL elements were manufactured according to the following procedure.

A glass substrate thin-film-coated with indium tin oxide (ITO) to a thickness of 1,200 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, HT-1 + 2% HAT-CN (100 Å) / HT-1 (1400 Å) / HT-2 (50 Å) / BH + 2% BD (200 Å) / ET-2 (50 Å) / each compounds 1, 3, 11, 27, 41, 57, 59, 81, 83, 84 : LiQ = 1:1 (300 Å) / LiF (10 Å ) / Al (1000 Å) were stacked in order to manufacture an organic EL element.

In such a case, the structures of HT-1, HAT-CN, HT-2, BH, BD, ET-1, ET-2 and LiQ are each as follows.

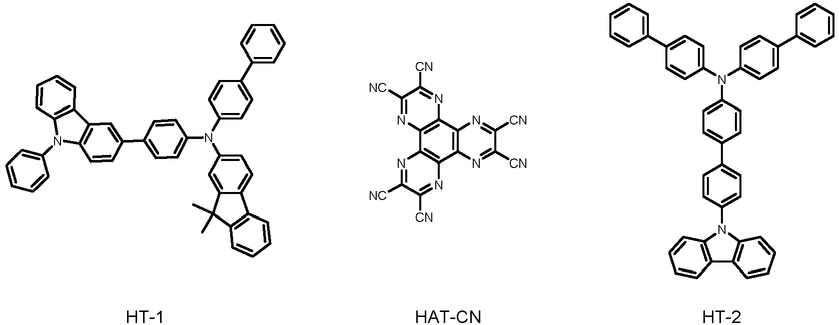

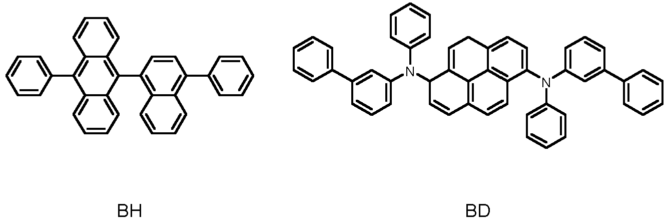

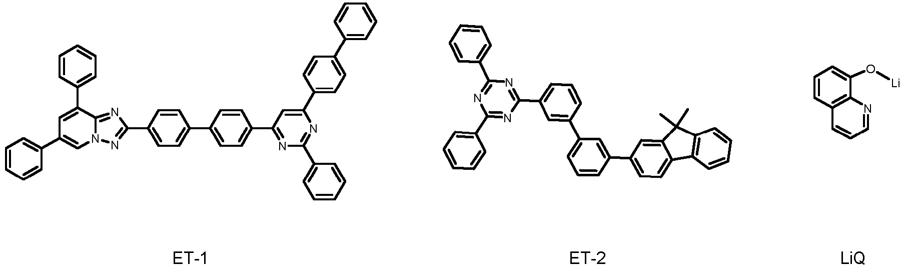

### [Comparative Example 1] Manufacturing of blue organic EL element

A blue organic EL element of Comparative Example 1 was manufactured in the same manner as in Example 1, except that Compound ET-1 was deposited at 30 nm instead of Compound 1 used as the electron transport layer material in Example 1.

### [Evaluation example 1]

For each of the blue organic EL elements manufactured in Examples (Ex.) 1 to 10 and Comparative Example (Comp. Ex.) 1, a driving voltage, an emission peak, and a current efficiency at a current density of 10 mA/cm² were measured, and the results are shown in Table 1 below.

**[Table 1]**

| Sample | Electron transport layer material | Driving voltage (V) | EL peak (nm) | Current efficiency (cd/A) |
|---|---|---|---|---|
| Ex. 1 | Compound 1 | 4.3 | 460 | 6.6 |
| Ex. 2 | Compound 3 | 4.6 | 461 | 6.7 |
| Ex. 4 | Compound 11 | 4.4 | 460 | 6.7 |
| Ex. 5 | Compound 27 | 4.5 | 461 | 6.5 |
| Ex. 6 | Compound 41 | 4.1 | 459 | 6.9 |
| Ex. 6 | Compound 57 | 4.2 | 458 | 6.5 |
| Ex. 7 | Compound 59 | 4.6 | 460 | 6.2 |
| Ex. 8 | Compound 81 | 4.1 | 462 | 7.0 |
| Ex. 9 | Compound 83 | 4.3 | 458 | 6.3 |
| Ex. 10 | Compound 84 | 4.3 | 461 | 6.2 |
| Comp. Ex. 1 | ET-1 | 4.7 | 460 | 6.0 |

As shown in Table 1, it was appreciated that the blue organic EL elements of Examples 1 to 10 using the compound according to the present invention as an electron transport layer material exhibit significantly excellent performance in terms of current efficiency, emission peak and driving voltage compared to the blue organic EL element of Comparative Example 1 using conventional compound ET-1 as an electron transport layer material.

### [Examples 11 to 28] Manufacturing of Blue organic EL elements

The compounds synthesized in the above synthesis examples were subjected to high-purity sublimation purification in a conventionally known method, and then blue organic EL elements were manufactured according to the following procedure.

A glass substrate thin-film-coated with indium tin oxide (ITO) to a thickness of 1,200 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, HT-1 + 2% HAT-CN (100 Å) / HT-1 (1400 Å) / HT-2 (50 Å) / BH + 2% BD (200 Å) / each compounds 6, 10, 23, 27, 37-39, 48, 51, 54, 57, 67, 72, 80-81, 89, 94-95 (50 Å) / ET-1 : LiQ = 1:1 (300 Å) / LiF (10 Å) / Al (1000 Å) were stacked in order to manufacture an organic EL element according to Examples 11 to 28.

### [Comparative Examples 2 to 4] Manufacturing of blue organic EL element

A blue organic EL element of Comparative Examples 2 to 4 were manufactured in the same manner as in Example 11, except that Compounds ET-2 to ET-4 was used instead of Compound 6 as the auxiliary electron transport layer material in Example 11.

In such a case, the structures of ET-2, ET-3 and ET-4 are each as follows.

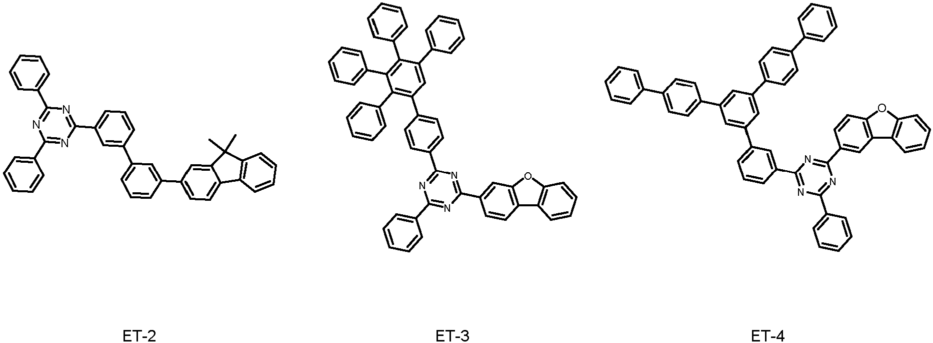

### [Evaluation example 2]

For each of the blue organic EL elements manufactured in Examples (Ex.) 11 to 28 and Comparative Examples (Comp. Ex.) 2 to 5, a driving voltage, an emission peak, and a current efficiency at a current density of 10 mA/cm² were measured, and the results are shown in Table 2 below.

**[Table 2]**

| Sample | Auxiliary electron transport layer material | Driving voltage (V) | EL peak (nm) | Current efficiency (cd/A) |
|---|---|---|---|---|
| Ex. 11 | Compound 6 | 4.1 | 460 | 6.7 |
| Ex. 12 | Compound 10 | 4.4 | 461 | 6.6 |
| Ex. 13 | Compound 23 | 4.0 | 462 | 7.0 |
| Ex. 14 | Compound 27 | 4.2 | 461 | 6.7 |
| Ex. 15 | Compound 37 | 3.9 | 460 | 7.1 |
| Ex. 16 | Compound 38 | 3.8 | 459 | 7.3 |
| Ex. 17 | Compound 39 | 4.4 | 458 | 6.3 |
| Ex. 18 | Compound 48 | 4.5 | 460 | 6.2 |
| Ex. 19 | Compound 51 | 4.0 | 462 | 7.2 |
| Ex. 20 | Compound 54 | 4.3 | 463 | 6.2 |
| Ex. 21 | Compound 57 | 4.1 | 458 | 6.3 |
| Ex. 22 | Compound 67 | 4.2 | 460 | 6.6 |
| Ex. 23 | Compound 72 | 3.8 | 461 | 7.2 |
| Ex. 24 | Compound 80 | 3.9 | 459 | 7.0 |
| Ex. 25 | Compound 81 | 4.2 | 460 | 6.8 |
| Ex. 26 | Compound 89 | 4.0 | 458 | 6.7 |
| Ex. 27 | Compound 94 | 3.9 | 459 | 6.9 |
| Ex. 28 | Compound 95 | 3.7 | 460 | 7.3 |
| Comp. Ex. 2 | ET-2 | 4.7 | 460 | 6.0 |
| Comp. Ex. 3 | ET-3 | 5.0 | 461 | 5.3 |
| Comp. Ex. 4 | ET-4 | 4.6 | 460 | 6.1 |

As shown in Table 2, it was appreciated that the blue organic EL elements of Examples 11 to 28 using the compound according to the present invention as an auxiliary electron transport layer material exhibit significantly excellent performance in terms of current efficiency, and driving voltage compared to the blue organic EL elements of Comparative Examples 2 to 4.

## Claims

1. A compound represented by Chemical Formula 1 below: 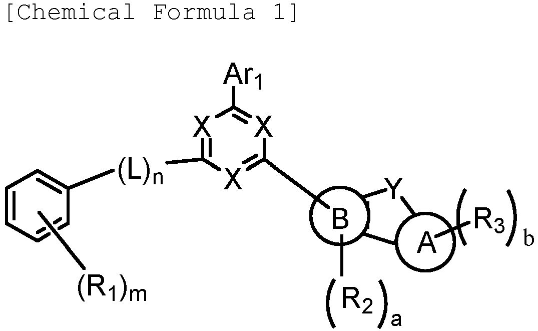 wherein Chemical Formula 1,
a plurality of X are the same or different from each other and are each independently CR₅ or N, with a proviso that at least two of the plurality of X are each N,
Ar₁ is selected from the group consisting of a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms,
Y is O, S, or NR₄,
Ring A and Ring B are the same as or different from each other, each independently being a C₅ to C₁₈ monocyclic or polycyclic hydrocarbon ring that may or may not include heteroatoms,
R₂ to R₃ are the same or different from each other and are each independently selected from the group consisting of a deuterium, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms, or combine with an adjacent group to form a fused ring;
R₄ to R₅ are same or different and are each independently selected from the group consisting of a hydrogen, a deuterium (D), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms;
L is selected from the group consisting of an arylene of C₆-C₁₈ and a heteroarylene of 5 to 18 nuclear atoms;
R₁ is selected from the group consisting of a C₆ to C₆₀ aryl group, a heteroaryl group having 5 to 60 nuclear atoms, and a C₆ to C₆₀ arylsilyl group;
m is an integer of 1 or greater, n is an integer of 0 to 3,
a is an integer of 0 to 2, b is an integer of 0 to 4, and
the arylene and heteroarylene groups of L, the aryl, heteroaryl, and arylsilyl groups of R₁, and the alkyl, alkenyl, alkynyl, cycloalkyl, heterocycloalkyl, aryl, heteroaryl, alkyloxy, aryloxy, alkylsilyl, arylsilyl, alkylborone, arylboron, arylphosphine, arylphosphine oxide, and arylamine groups of R₂ to R₅ and Ar₁ are optionally each independently unsubstituted or substituted with at least one selected from the group consisting of a hydrogen, a deuterium (D), a halogen, a cyano group, a nitro group, a C₁ to C₄₀ alkyl group, a C₂ to C₄₀ alkenyl group, a C₂ to C₄₀ alkynyl group, a C₃to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₁ to C₄₀ arylphosphine group, a C₆ to C₆₀ aryl phosphine oxide group, a C₆ to C₆₀ arylamine group, a C₅ to C₆₀ arylheteroarylamine group, and a heteroarylamine group having 5 to 60 nuclear atoms, and wherein when the substituent is present in a plural number, they are optionally the same or different from each other, and
provided that the total number of carbon atoms contained in Y-containing ring substituted or unsubstituted with the substituents comprises at least 16.

2. The compound of Claim 1, wherein R₁ is selected from the group consisting of a C₆ to C₃₀ aryl group, a heteroaryl group having 5 to 30 nuclear atoms, and a C₆ to C₃₀ arylsilyl group,
m is an integer of 1 to 5, and
the substituted or unsubstituted (R₁)ₘ has a total of at least 18 carbon atoms.

3. The compound of Claim 1, wherein the Y-containing ring is selected from the group of substituents represented by the following formulas: 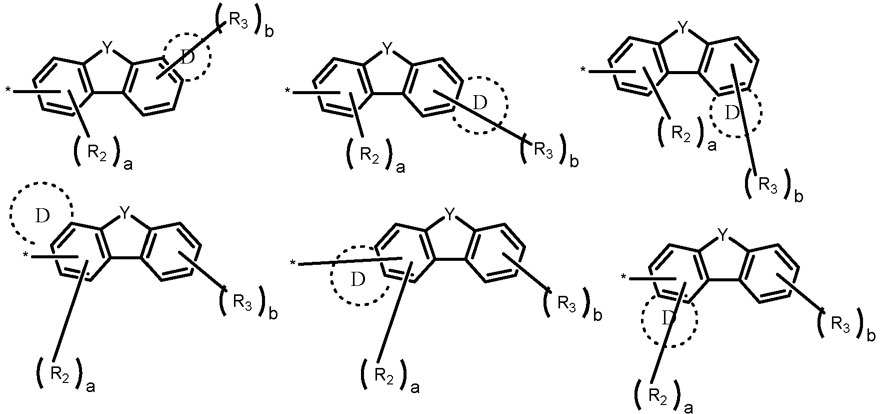 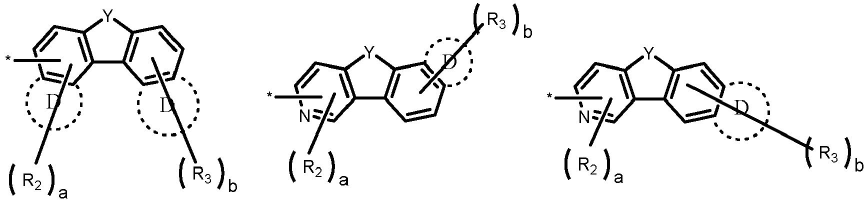 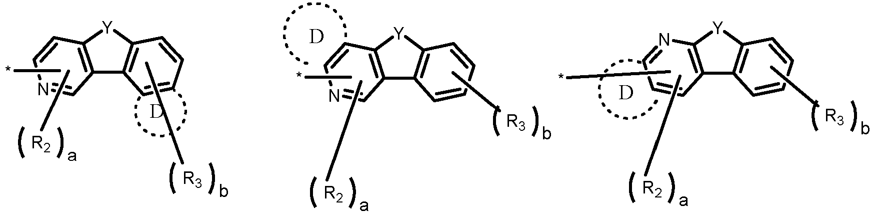 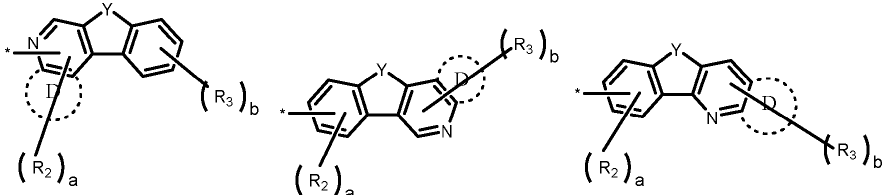 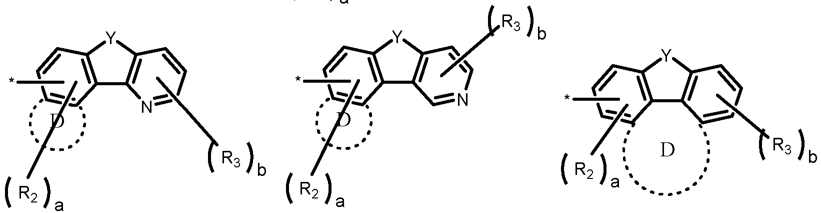 wherein the formulas,
* indicates a site where a bond with Chemical Formula 1 is made,
Ring D is the same or different from each other, and is each independently a monocyclic or polycyclic hydrocarbon ring that may or may not include heteroatoms,
Y, R₂, R₃, a and b are each as defined in claim 1.

4. The compound of Claim 1, wherein the Y-containing ring is selected from the group of substituents represented by the following formulas: 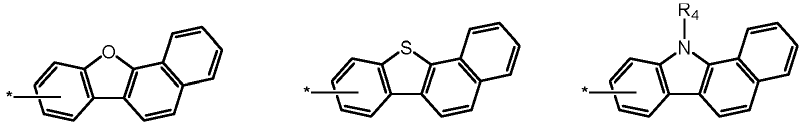 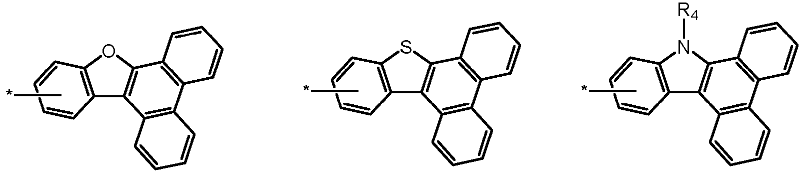 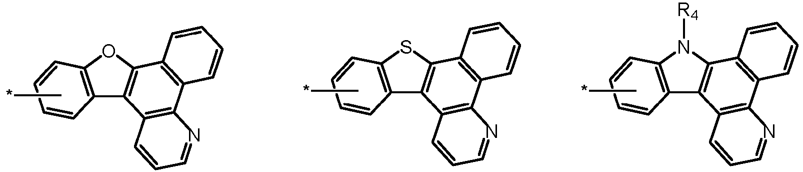 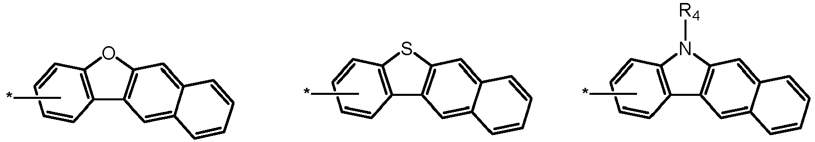 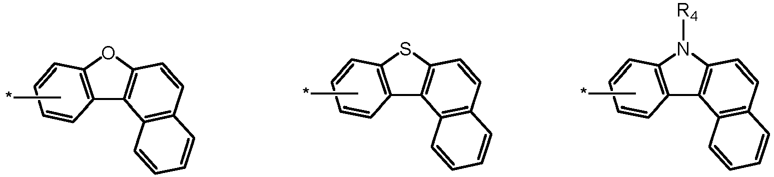 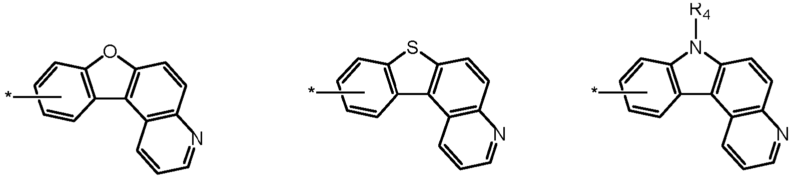 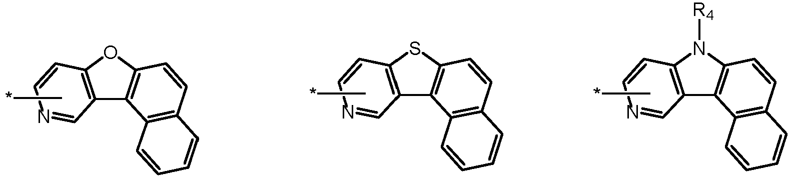 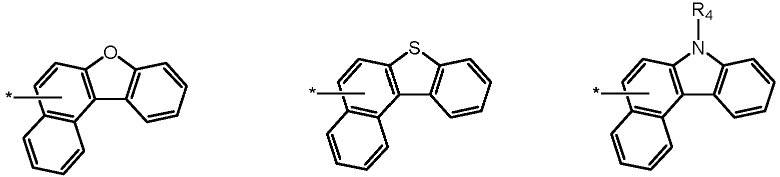 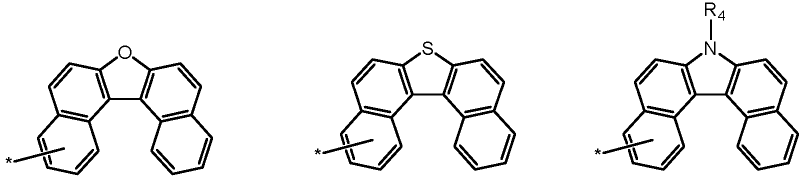 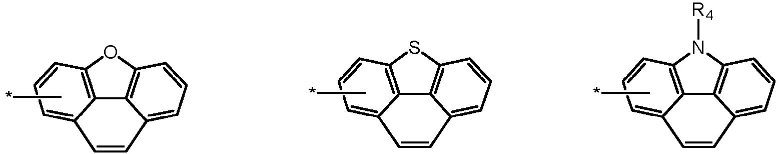 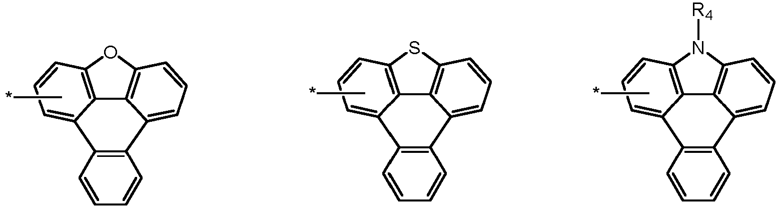 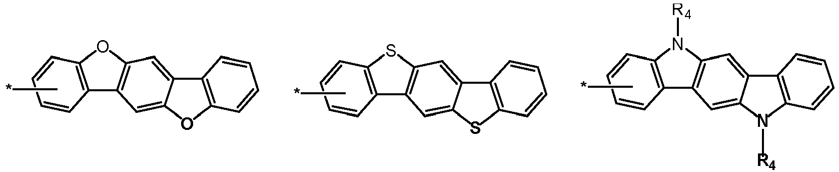 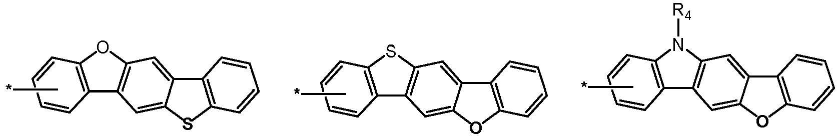 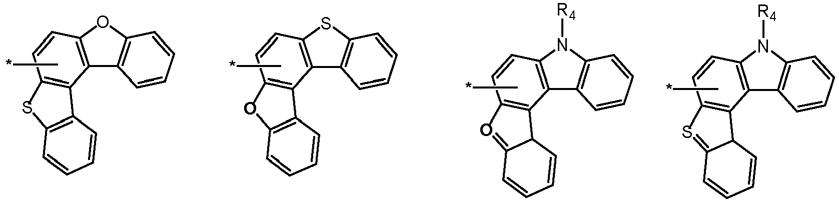 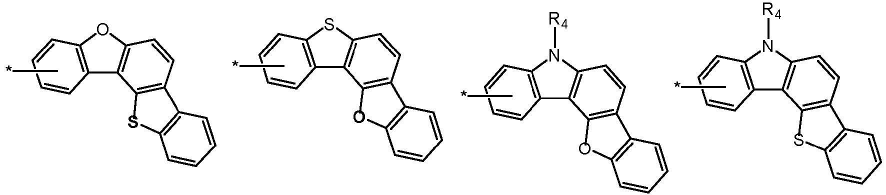 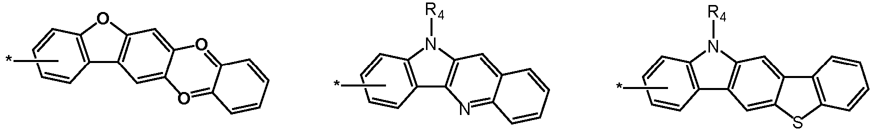 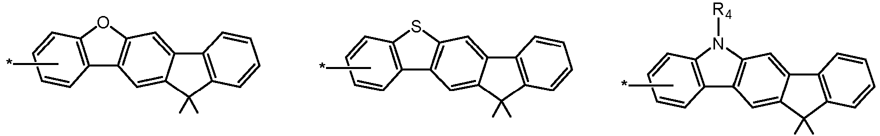 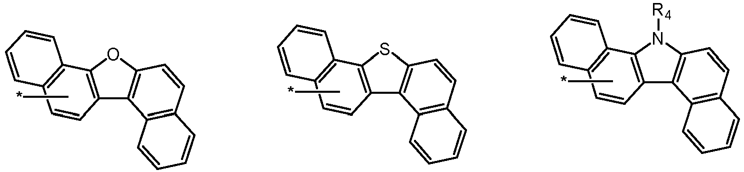 wherein the formulas,
* indicates a site where a bond with Chemical Formula 1 is made,
R₄ is as defined in claim 1.

5. The compound of claim 1, wherein the compound represented by Chemical Formula 1 is a compound represented by any one of Chemical Formulas 2 to Chemical Formula 5 below: 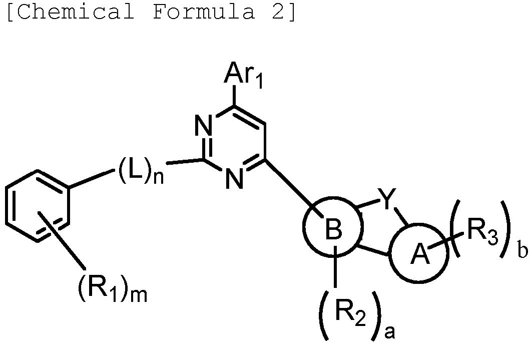 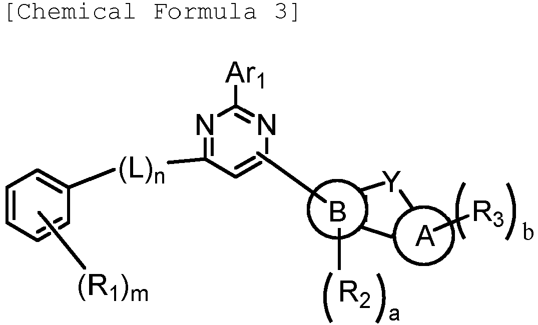 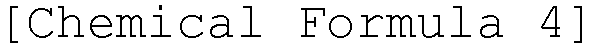 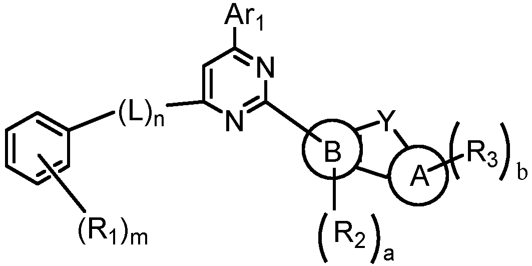 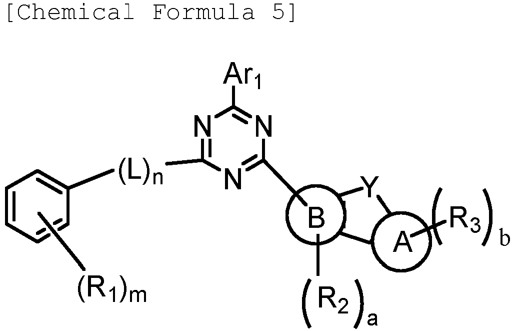 wherein the formulas,
* indicates a site where a bond with Chemical Formula 1 is made,
A, B, Y, L, R₁~R₃, Ar₁, a, b, m and n are each as defined in claim 1.

6. The compound of claim 1, wherein Ar₁ and R₂ to R₃ are the same or different from each other and are each independently selected from the group consisting of a C₆ to C₆₀ aryl group, and a heteroaryl group having 5 to 60 nuclear atoms,
R₄ is selected from the group consisting of a C₆ to C₆₀ aryl group, and a heteroaryl group having 5 to 60 nuclear atoms.

7. The compound of claim 1, wherein Ar₁ is selected from the group of substituents represented by the following formula:

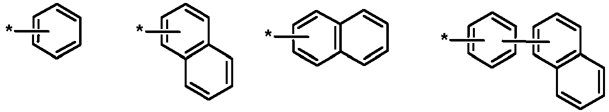

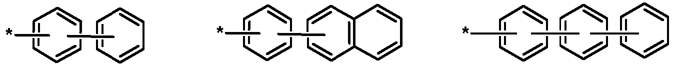

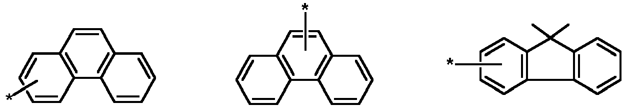

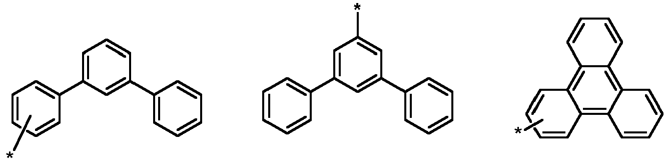

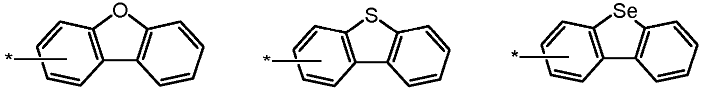

8. The compound of claim 1, wherein the compound represented by Chemical Formula 1 is a compound represented by any one of Chemical Formulas 6 to Chemical Formula 13 below: 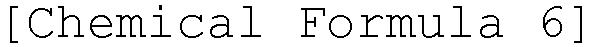 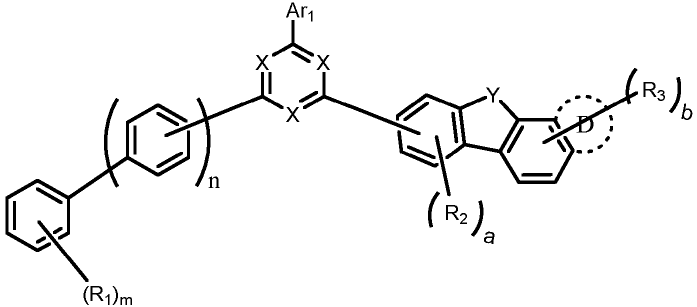 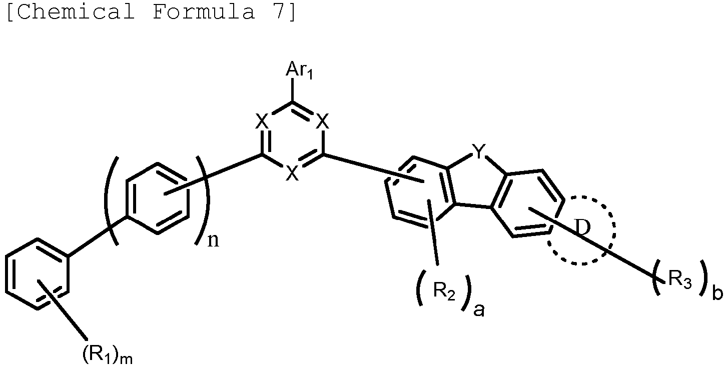 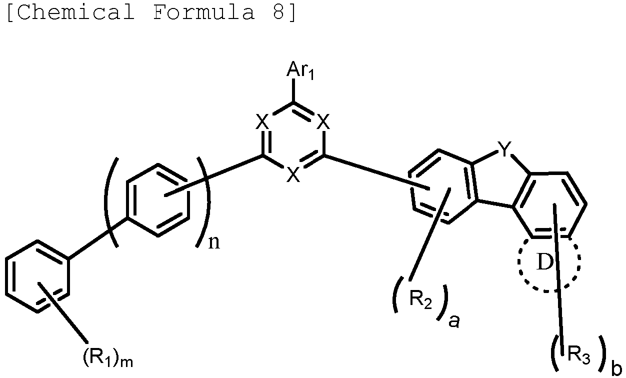 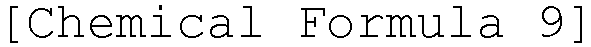 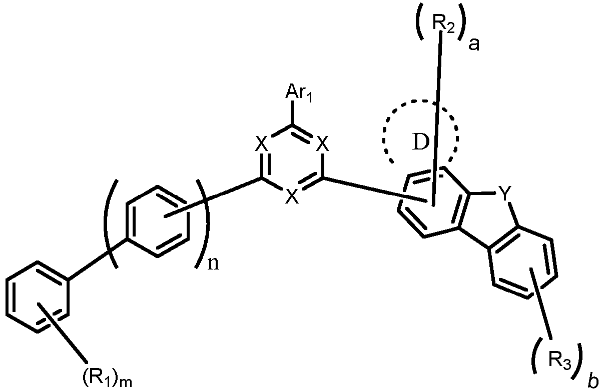 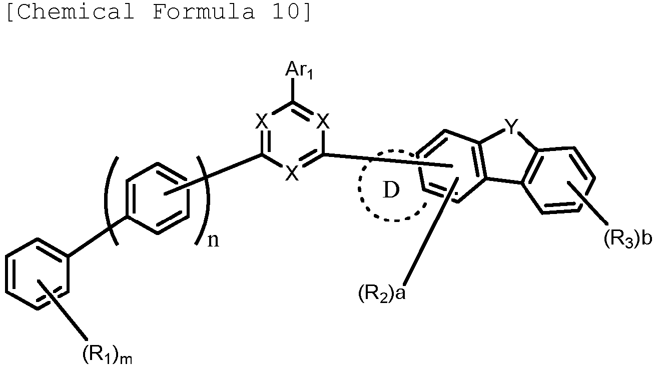 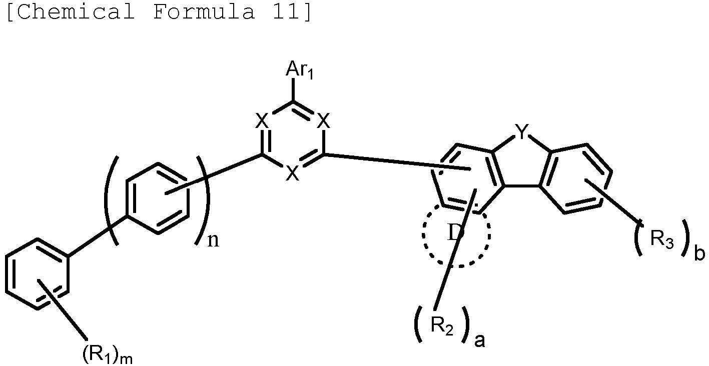 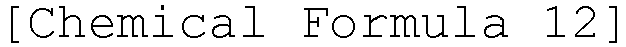 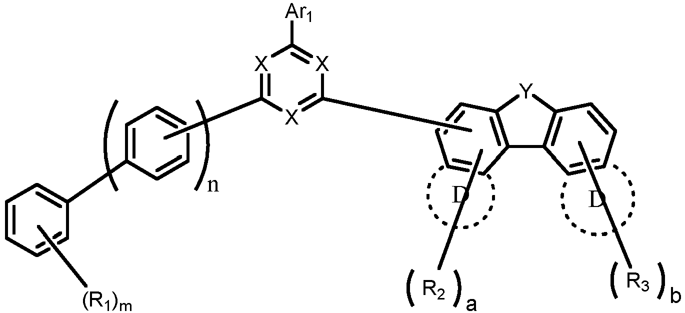 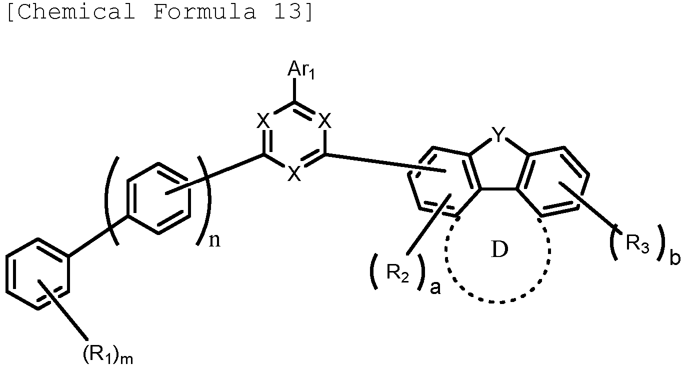 wherein the formulas,
Ring D is a monocyclic or polycyclic hydrocarbon ring group containing or not containing a heteroatom,
X, Y, R₁~R₃, Ar₁, a, b, m and n are each as defined in claim 1.

9. The compound of claim 1, wherein the compound represented by Chemical Formula 1 is a compound represented by any one of Chemical Formulas 14 to Chemical Formula 25 below: 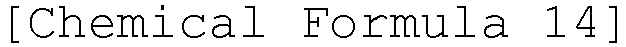 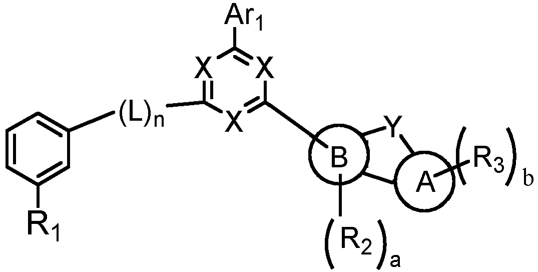 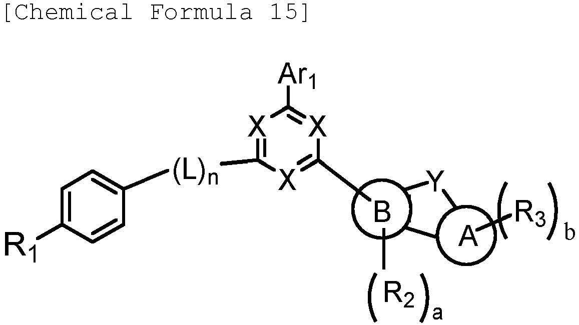 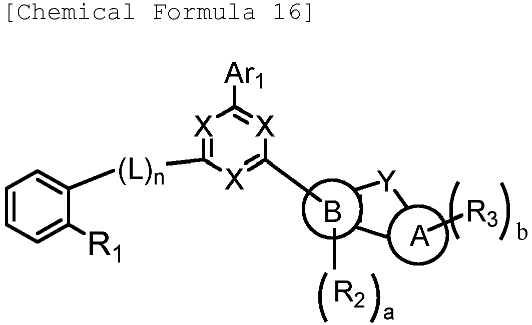 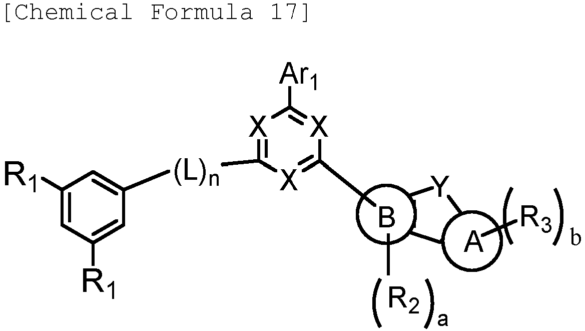 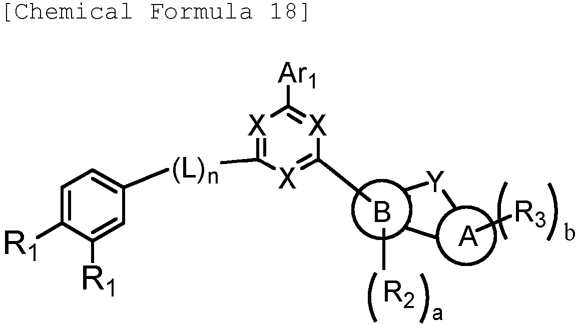 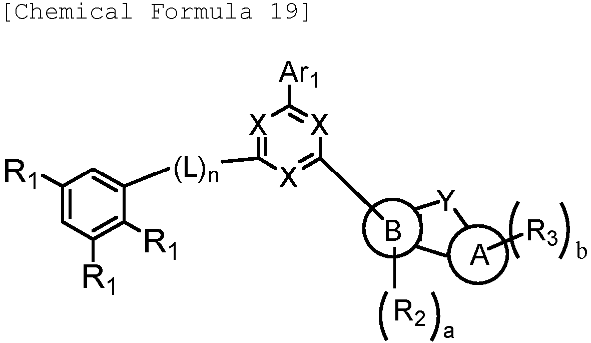 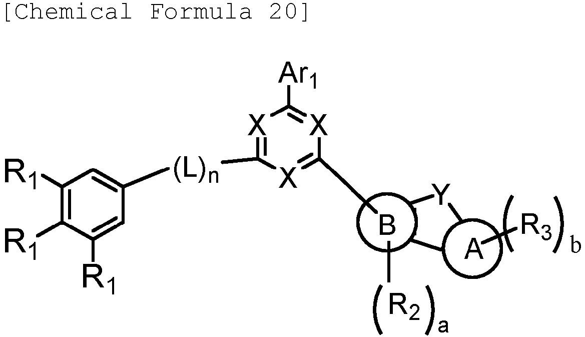 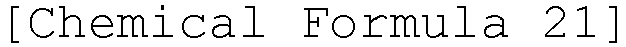 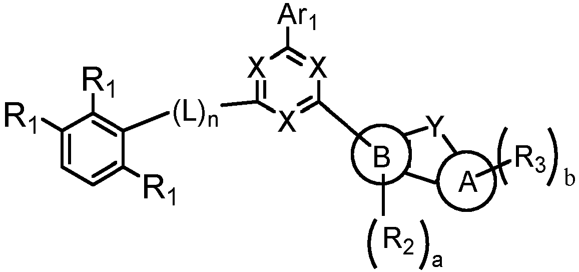 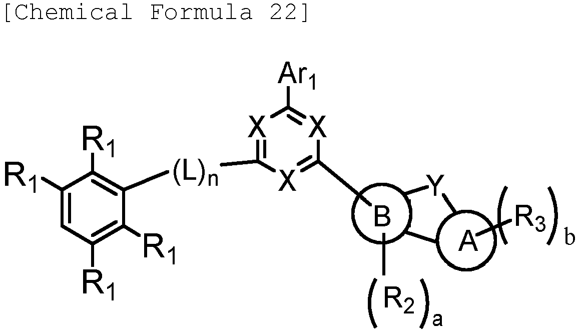 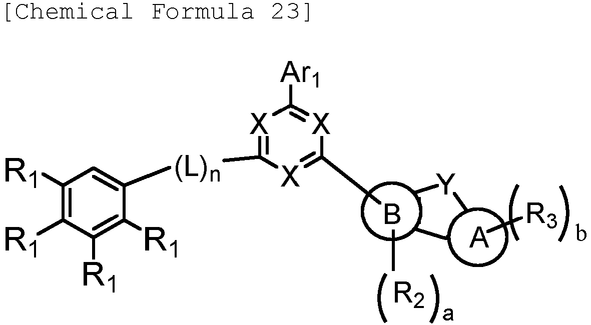 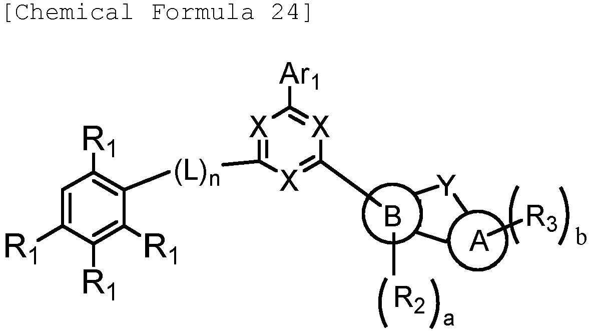 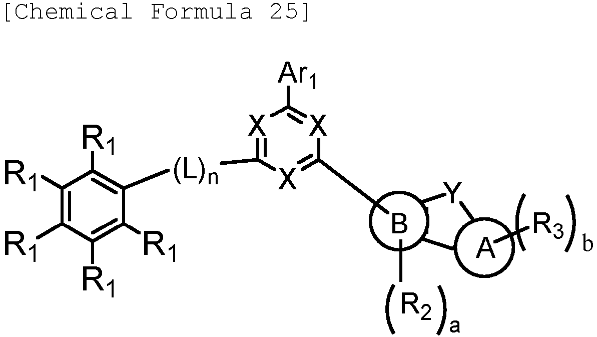 wherein the formulas,
X, Y, A, B, Ar₁, L, R₁~R₃, a, b and n are each as defined in claim 1.

10. The compound of claim 1, wherein the compound represented by Chemical Formula 1 is selected from compounds represented by any one of the following Chemical Formulas 1 to 108.

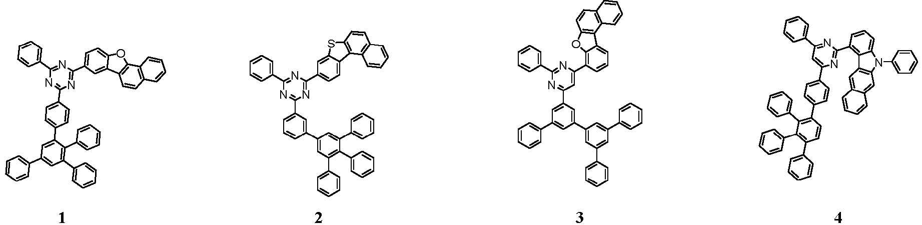

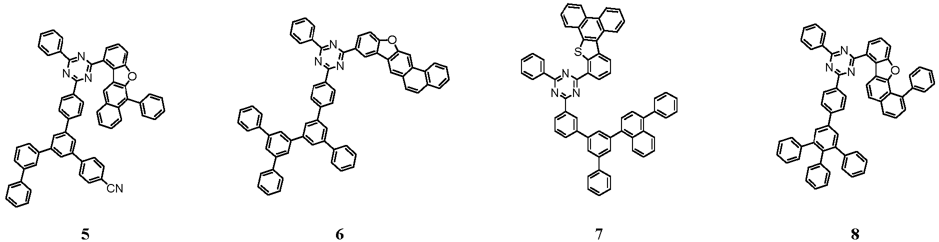

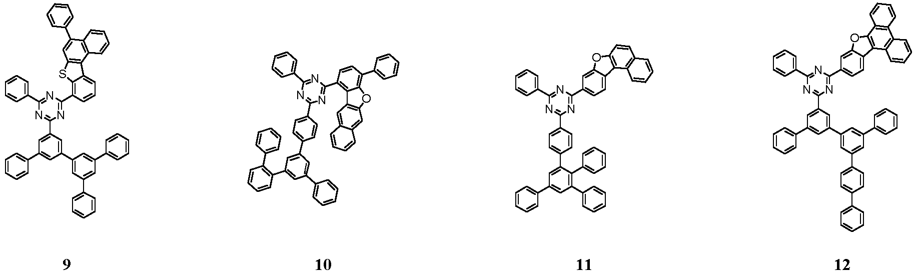

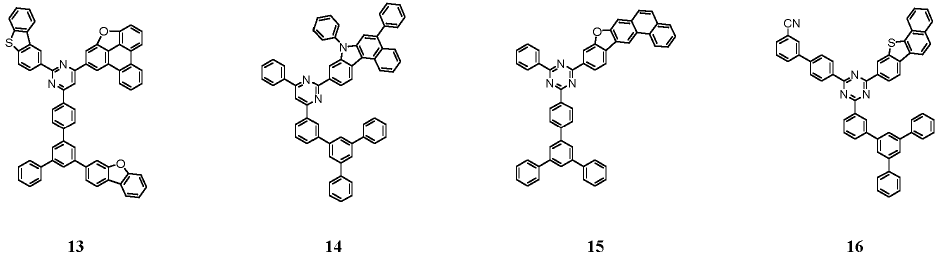

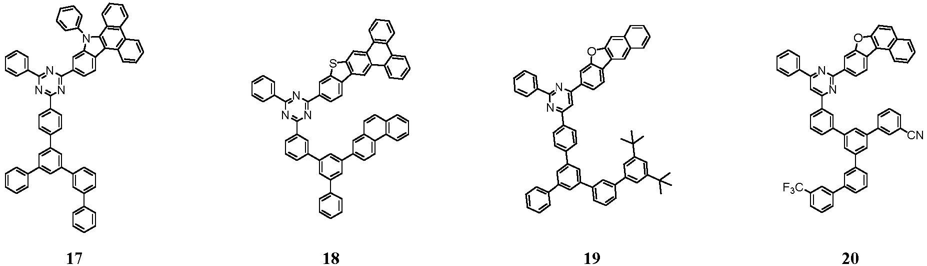

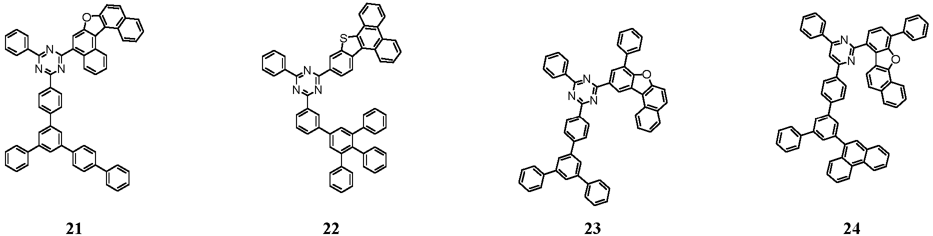

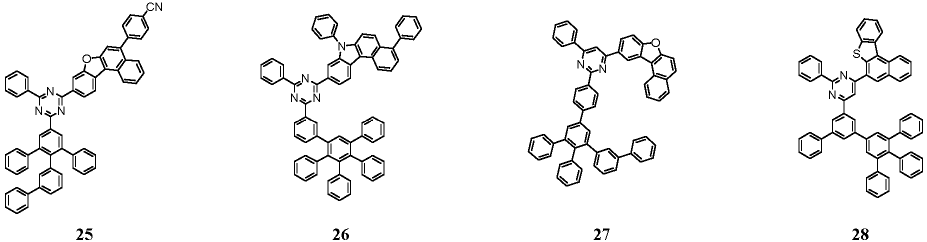

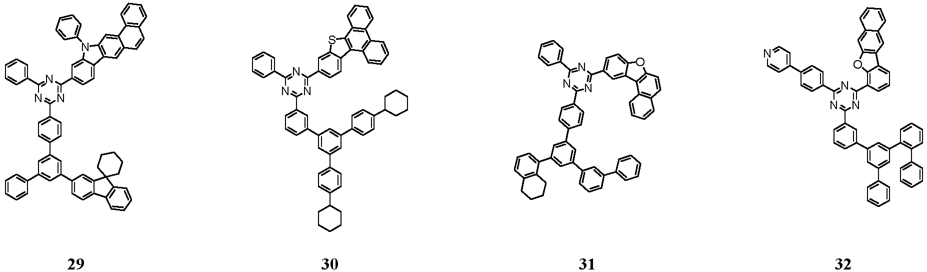

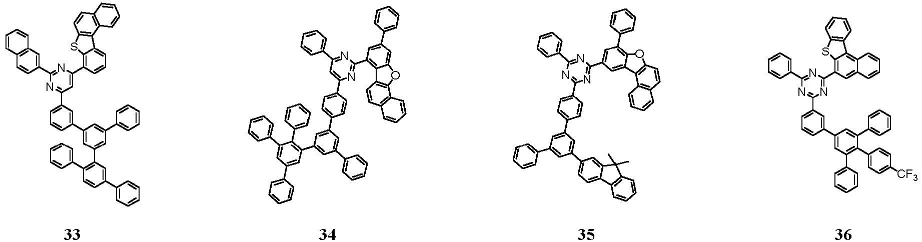

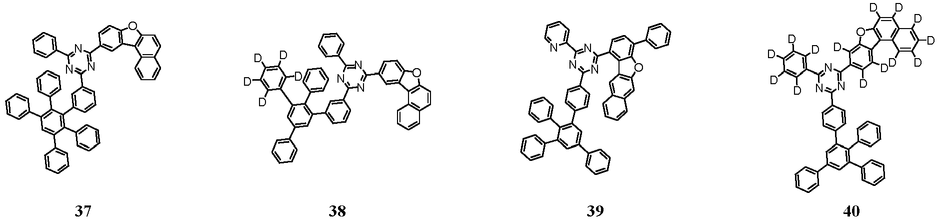

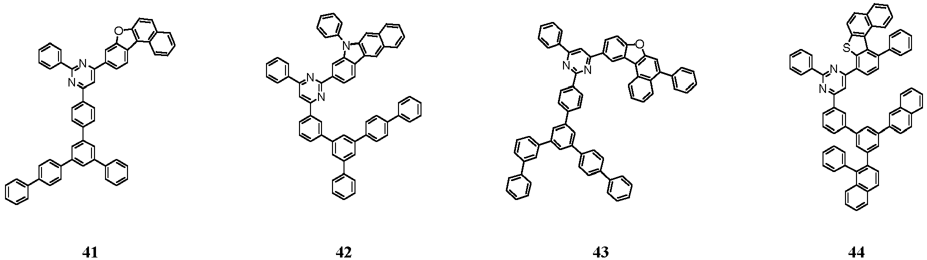

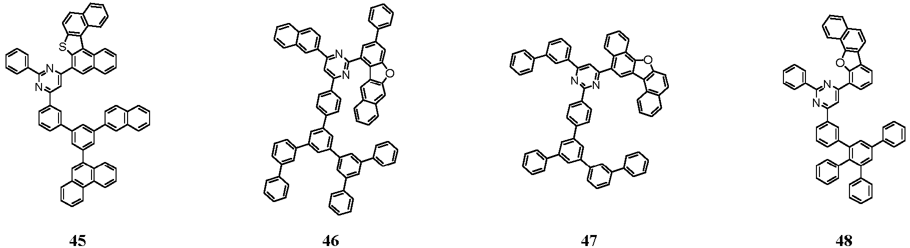

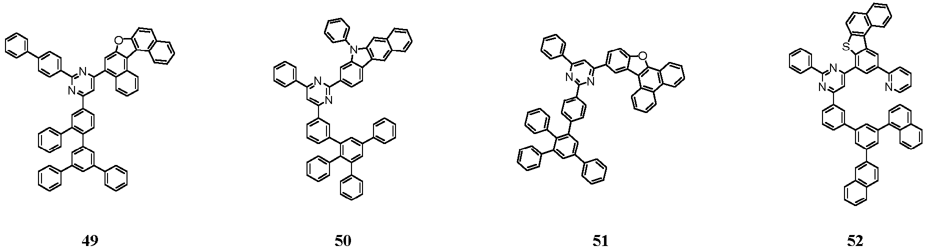

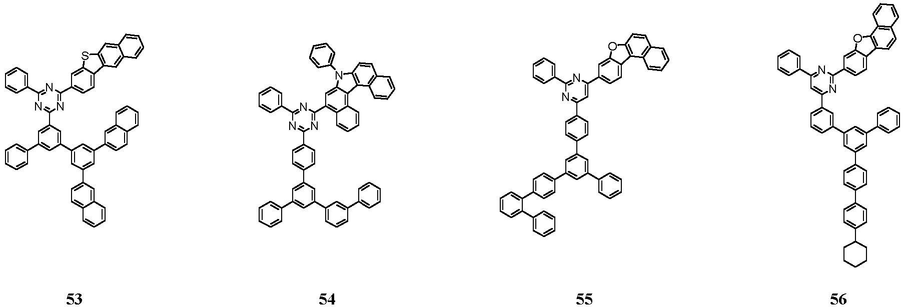

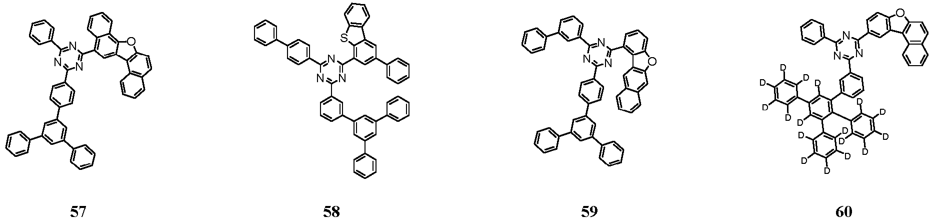

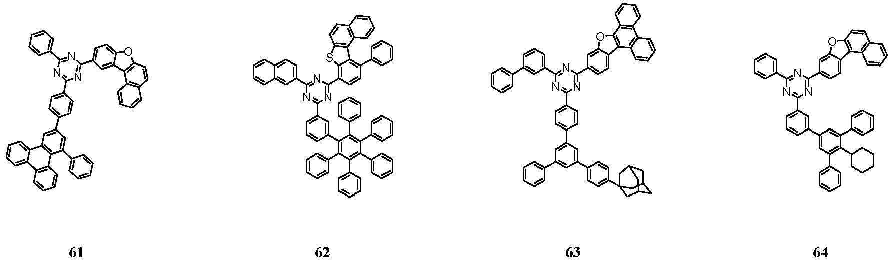

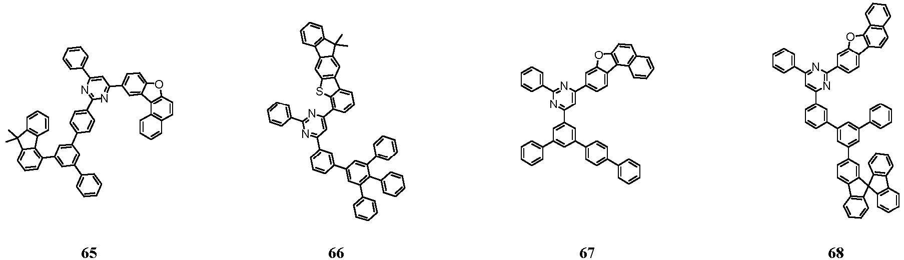

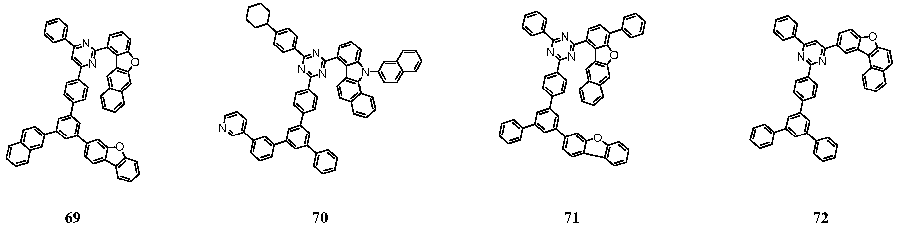

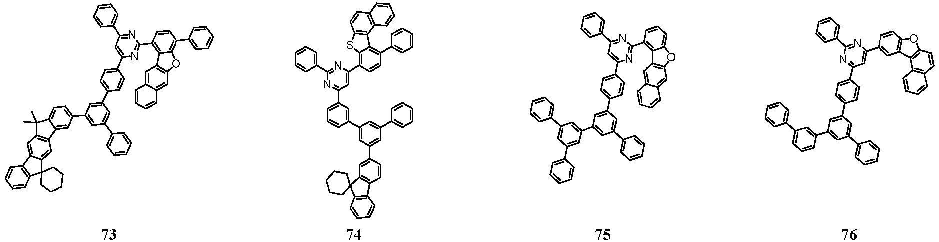

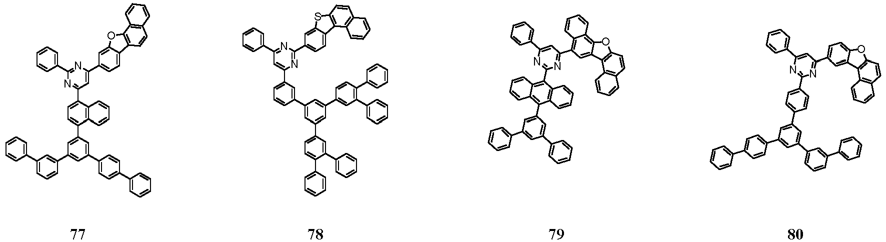

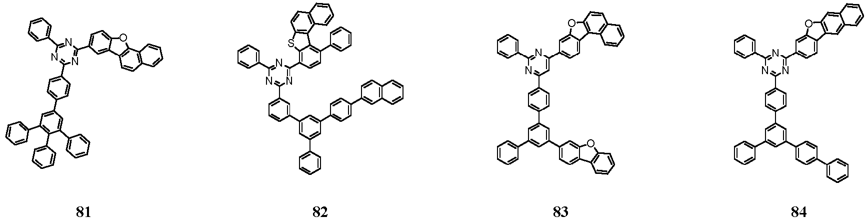

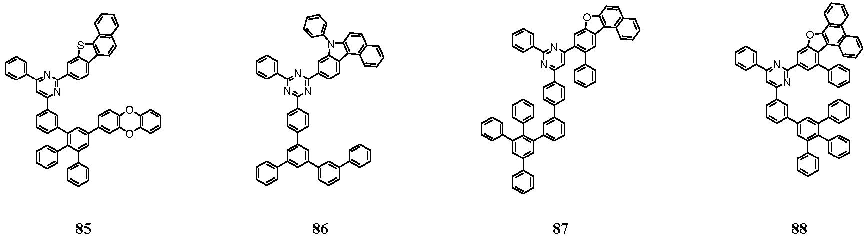

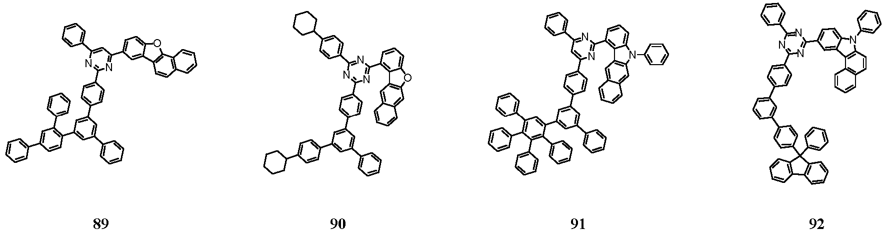

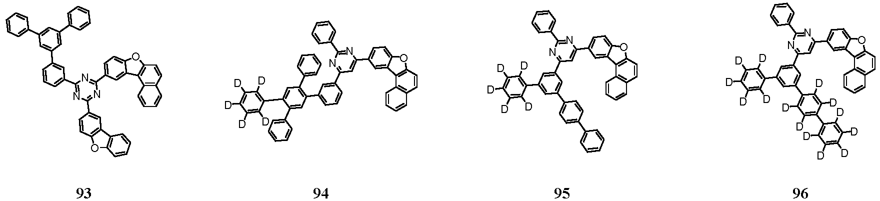

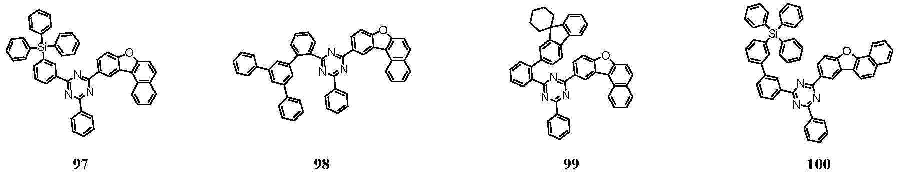

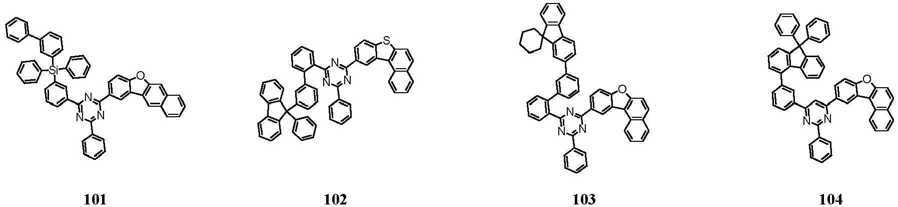

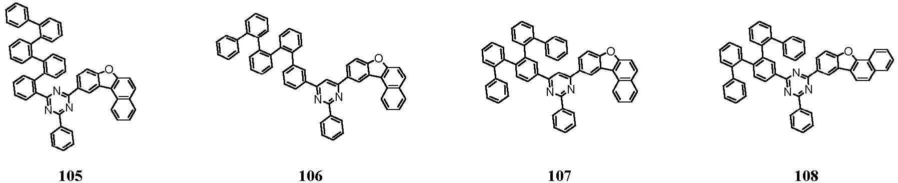

11. The compound of claim 1, wherein the compound represented by Chemical Formula 1 is used as a material of a light-emitting layer, an electron transport layer, or an electron transport auxiliary layer.

12. An organic electroluminescent device comprising:
an anode;
a cathode; and
one or more organic layers disposed between the anode and the cathode,
wherein at least one of the one or more organic layers comprises the compound according to any one of claims 1 to 11.

13. The organic electroluminescent device of claim 12, wherein the organic layer comprising the compound is selected from: a light-emitting layer, a light-emitting auxiliary layer, a hole injection layer, a hole transport layer, an electron injection layer, a life-improving layer, an electron transport layer, and an electron transport auxiliary layer.

14. The organic electroluminescent device of claim 13, wherein the compound is included as at least one of a phosphorescent host material of a light emitting layer, an electron transport layer, and an electron transport auxiliary layer.
